# EUROPEAN PATENT SPECIFICATION

(11) **EP 2 780 318 B1**
(45) Date of publication and mention of the grant of the patent: **26.08.2015**
(21) Application number: 12784603.8
(22) Date of filing: 14.11.2012
(51) Int. Cl.: C07C 237/22

(54) **PROCESS FOR THE PRODUCTION OF N-SUBSTITUTED 2-(ACETYLAMINO)-N'-BENZYL-3-METHOXYPROPANAMIDES**
VERFAHREN ZUR HERSTELLUNG VON N-SUBSTITUIERTEN 2-(ACETYLAMINO)-N'-BENZYL-3-METHOXYPROPANAMIDEN
PROCÉDÉ POUR LA PRODUCTION DE 2-(ACÉTYLAMINO)-N'-BENZYL-3-MÉTHOXYPROPANAMIDES N-SUBSTITUÉS

(30) Priority: 15.11.2011 EP 11009048
(43) Date of publication of application: 24.09.2014
(73) Proprietor: Sanofi-Aventis Deutschland GmbH, 65929 Frankfurt am Main (DE); Zentiva, k.s., Dolni Mecholupy 102 37 Praha 10 (CZ)
(72) Inventor: WEHLAN, Hermut, 65926 Frankfurt am Main (DE); ROSSEN, Kai, 65926 Frankfurt am Main (DE); OEHME, Jan, 65926 Frankfurt am Main (DE); KRAL, Vladimir, 120 00 Praha 2 (CZ)
(74) Representative: Jirotkova, Ivana
(86) International application number: PCT/EP2012/072548
(87) International publication number: WO 2013/072330

(56) References cited:
- WO-A2-2010/107993
- US-A1- 2009 143 472

## Description

### Technical field

The present invention relates to a chemical process for the manufacture of N-substituted 2-(acetylamino)-N'-benzyl-3-methoxypropanamides of the formula I and their use as intermediates in the production of drugs. For instance, (R)-2-{Acetyt-[1-(4-methoxy-phenyl)-ethyl]-amino}-N-benzyl-3-methoxy-propionamide of the formula I, wherein R1 is 1-(4-methoxy-phenyl)-ethyl is a key intermediate for the production of (+)-(2R)-2-(acetylamino)-N-benzyl-3-methoxypropanamide of the formula II ((R)-Lacosamide).

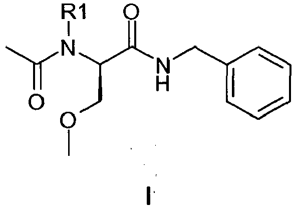

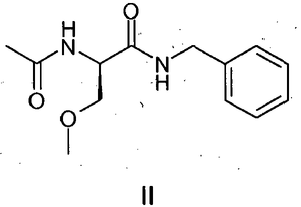

### Background art

(R)-Lacosamide II is an anticonvulsant drug for the treatment of epileptic seizures (US5773465) and several prior art methods are disclosed for its preparation. These methods involve stepwise procedures via a number of intermediates (Scheme 1), starting from D-Serine (R2 = H) or *N*-protected D-Serine of formula III, which were double alkylated to gave the intermediate ester IV. Ester IV were converted into the benzyl amide V by a 3-step sequence; consisting of ester hydrolysis, acid activation / amid coupling and protecting group (R2) cleavage. (R)-Lacosamide II was obtained by final N-acetylation of the intermediate V.

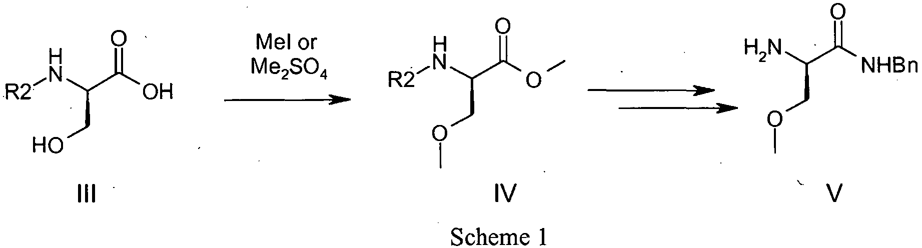

The intermediate of the formula III is the starting point for a number of processes in the synthesis of (*R*)-Lacosamide. For instance: the use of R2 = benzyloxycarbonyl (CbZ) was described by H. Kohn in US5772475, for R2 = tert-butoxycarbonyl (Boc) by Riedner in EP1642889 and for R = triphenylmethyl (trityl) in US20090143472.

Furthermore, intermediates of formula VI, where R2 is a protecting group as described for Scheme 1 and R3 is a hydroxyl protecting group, like benzyl, tertiary butyl or benzoyl are disclosed in WO2011/039781 and in WO2011/0156617 (for R3 = tetrahydropyranyl). Compounds of formula VI were converted into Lacosamide by a multi step sequence, consisting of the removal of R3, *O*-methylation, removal of R2 and *N*-acetylation.

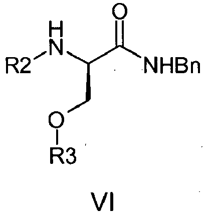

All intermediates III-VI used in the synthesis of (R)-Lacosamide made use of the unnatural and expensive D-Serine III (R2 = H), which was manipulated by several *N*/*O*-protection- deprotection sequences, leading in a 5-6 step synthesis to Lacosamide.

WO 2010/107993 describes the O-methylation via dimethylsulfate and a N-methylated derivative of lacosamide which is a by-product if certain other reaction conditions are used for O-methylation during the synthesis of lacosamide.

### Disclosure of invention

In a first aspect the present invention relates to a compound of formula I, enantiomerically and diastereomerically pure or as mixture with the corresponding enantiomer or diastereomer, whereas formula I represents both the pure enantiomer or diastereomer as well as its mixture with the corresponding enantiomer or diastereomer, 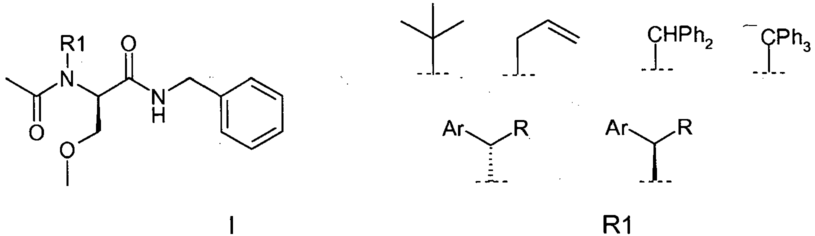 wherein
R1 is allyl-, tert-butyl-, di- and triphenylmethyl or
1-aryl-1-R-methyl group wherein
aryl (Ar) is a phenyl group, optionally substituted by one, two or three residues independently selected from (C₁-C₄)alkyl, (C₁-C₄)alkoxy, halogen or nitro; and
R is H or a (C₁-C₄)alkyl group with n = 1-4 carbon atoms - wherein for n = 2, 3 or 4 R can be connected to the aryl group to form an aromatic or partially unsaturated C₆₋C₁₀ bicyclic residue.

Halogen means fluoro (F), chlor (Cl), bromo (Br) or iodo (I), preferably F or Cl.

In one embodiment an R1 group, wherein R is H, is selected from benzyl, 2-methoxy benzyl, 3-methoxy benzyl, 4-methoxy benzyl, 3,4-dimethoxy benzyl or 4-methoxybenzyl. In another embodiment of R1, wherein R is alkyl, R1 is selected from 1-(4-methoxy-phenyl)-ethyl, 1-phenyl-ethyl, 1-phenyl-propyl, 1-(4-methyl-phenyl)-ethyl, 1-(2-methoxy-phenyl)-ethyl, 1-(4-nitro-phenyl)-ethyl, indan-1-yl, or 1-(1,2,3,4-tetrahydro-naphthalen-1-yl)-ethyl, which is of either R or S-configuration or a mixture thereof,

In yet another embodiment of R1, R1 is selected from 1-naphthalen-2-yl-ethyl, and 1-naphthalen-1-yl-ethyl.

In a further aspect the present invention provides a process for the preparation of (R)-Lacosamide (formula II) starting either from commercially available materials or compounds described already in the literature, themselves being prepared easily from commercially available materials, by using simple and environmentally compatible reagents and solvents, to afford high overall yields and good purity of the products.

This is achieved in accordance with the present invention - which, in one aspect thereof, provides a process for preparing a novel intermediate I, which, in a further aspect, can be further converted into (*R*)-Lacosamide II, starting with commercially available compounds such as acetic acid, methoxyacetaldehyde, benzyl isonitrile, as well as a chiral amines and salts thereof.

Thus, in one aspect the invention relates to a process for preparing a compound of formula I, enantiomerically and diastereomerically pure or as mixture with the corresponding enantiomer or diastereomer, whereas formula I represents both the pure enantiomer or diastereomer as well as its mixture with the corresponding enantiomer or diastereomer, 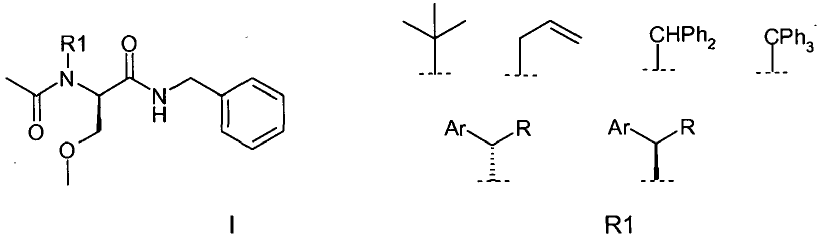 which comprises 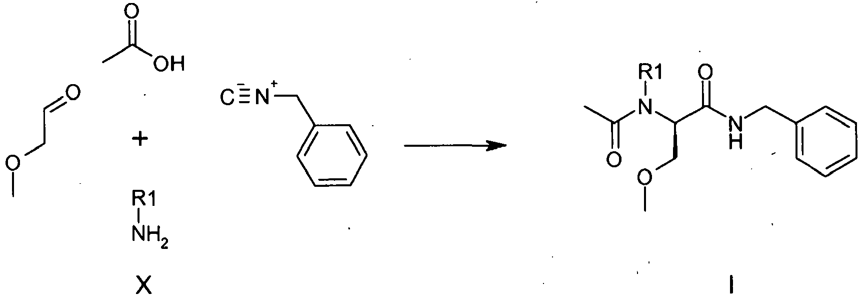 reacting a compound R1-NH2 (X) with acetic acid, Methoxyacetaldehyde and Benzylisonitrile, wherein
R1 is allyl-, tert-butyl-, di- and triphenylmethyl or is
a 1-aryl-1-R-methyl-group wherein
aryl (Ar) is a phenyl group, optionally substituted by one, two or three residues, preferably one or two, independently selected from (C₁-C₄)alkyl, (C₁-C₄)alkoxy, halogen or nitro; and
R is H or a (C₁-C₄)alkyl group with n = 1-4 carbon atoms - wherein for n = 2, 3 or 4 R can be connected to the aryl group to form an aromatic or partially unsaturated C₆-C₁₀ bicyclic residue.

Optionally, compound I is separated from its enantiomer Ia (if R1 is achiral) or diastereomer (if R1 is chiral) la 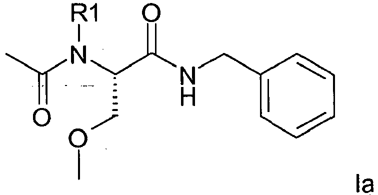 as described below for obtaining compound I in enantiomerically or diasteriomerically pure form.

The reaction of the four components with each other is variable and can be performed in various manners to yield a compound of formula I. The four components may be mixed simultaneously or are added successively in a certain order.

In one embodiment (method 1) of this aspect the present invention relates to a process for preparing a compound of the formula I 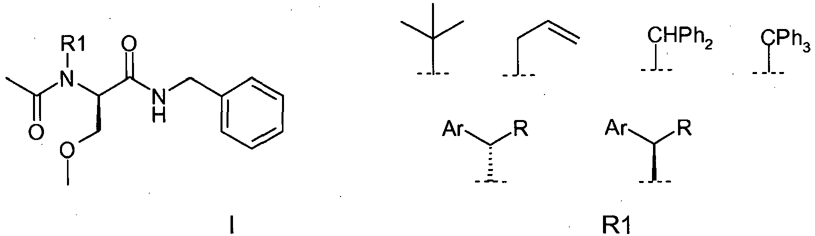 which comprises, as shown in scheme 2, 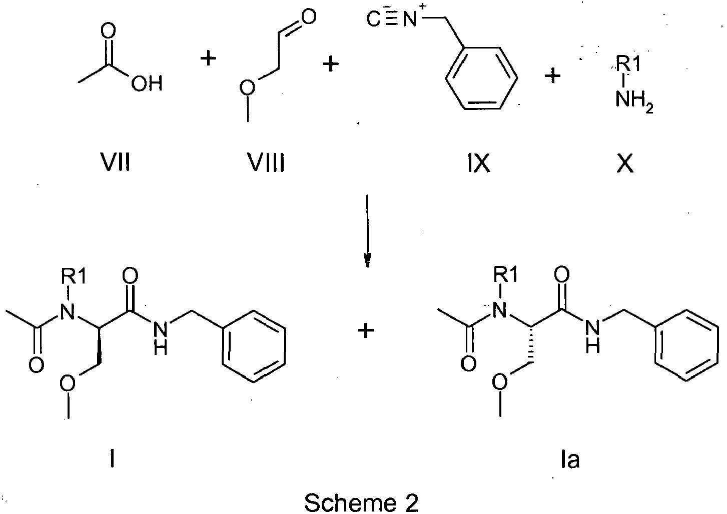 adding an amine R1 NH2 (X) where R1 is defined as in formula I above, to a mixture of acetic acid (VII), methoxyacetaldehyde (VIII) and benzylisonitrile (IX)
yielding a compound of formula I and Ia.

The mixture of acetic acid (VII), methoxyacetaldehyde (VIII) and benzylisonitrile (IX) may be prepared by mixing the components simultaneously or successively.

In a further embodiment (method 2) of this aspect the present invention relates to a process for preparing a compound of formula I 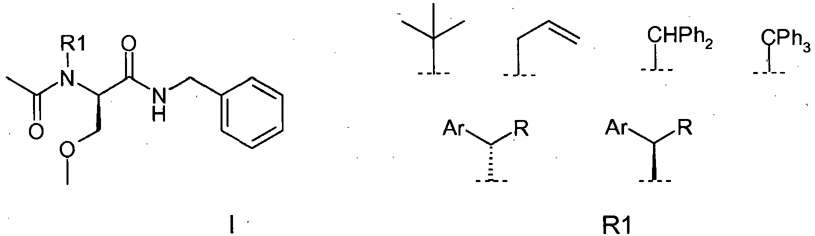 which comprises, as shown in scheme 3, 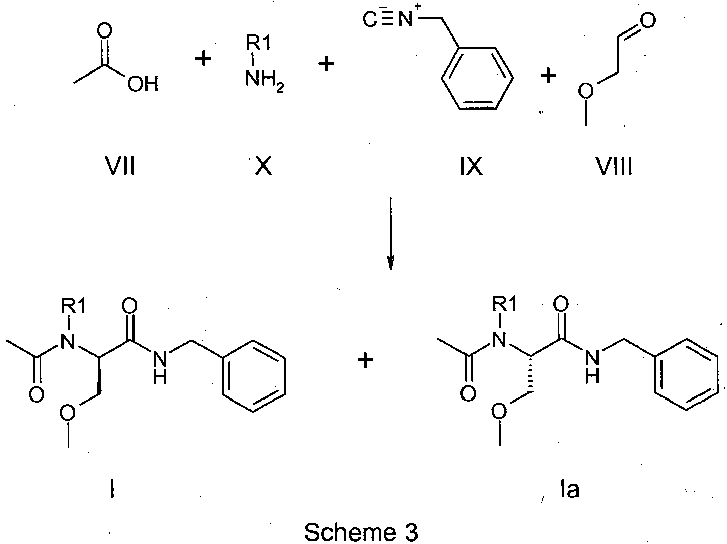 adding methoxyacetaldehyde (VIII) to a mixture of an amine R1 NH2 (X) wherein R1 is defined as in formula I, acetic acid and benzylisonitrile yielding a compound of formula I and Ia.

In another embodiment, the amines of formula X and acetic acid of formula XII can be mixed together, to obtain a salt of formula XI, prior to their addition to the reaction mixture. Also methoxyacetaldehyde of formula VIII can be added separately to the reaction mixture.

Therefore, in a further embodiment (method 3) the present invention also relates to a process for preparing a compound of formula I 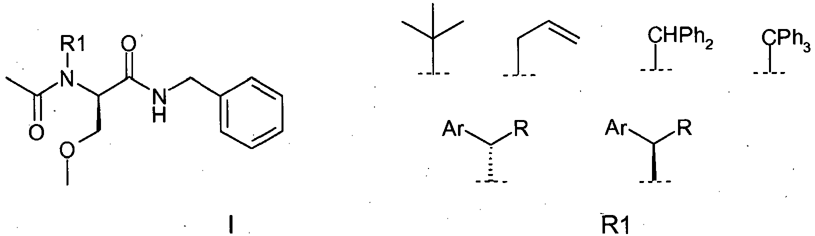 which comprises, as shown in scheme 4, 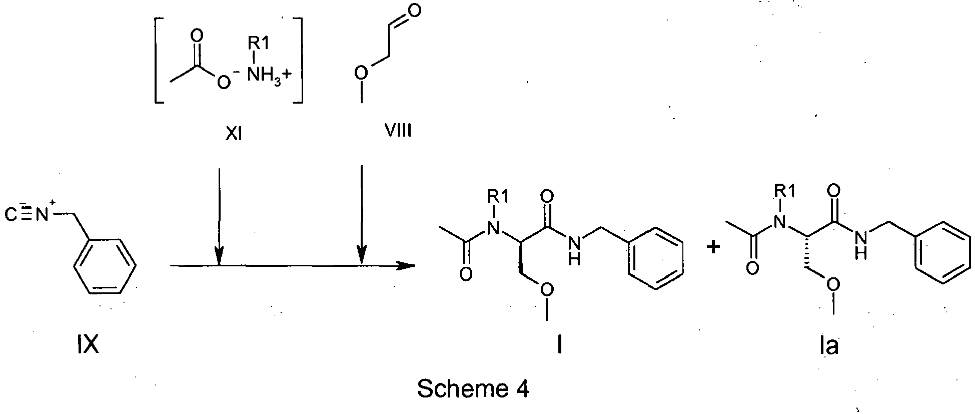 adding a salt (XI), prepared by mixing a compound R1 NH2 (X) and acetic acid, and methoxyacetaldehyde simultaneously or successively to benzylisonitrile,
yielding a compound of formula I and Ia.

In one embodiment of the compound of formula I prepared according to any of the above methods 1, 2 or 3, R1 is a 1-aryl-1-R-methyl group - where aryl (Ar) is a phenyl group, optionally substituted by one, two or three residues independently selected from (C₁-C₄)alkyl, (C₁-C₄)alkoxy, halogen or nitro; and R is H or a (C₁-C₄)alkyl group with n = 1-4 carbon atoms - wherein for n = 2, 3 or 4 R can be connected to the aryl group to form an aromatic or partially unsaturated C₆-C₁₀ bicyclic residue.

In one embodiment of the above methods 1, 2 or 3, where R in R1 is (C₁-C₄)alkyl, the R1 group is selected from 1-(4-methoxy-phenyl)-ethyl, 1-phenyl-ethyl, 1-phenyl-propyl, 1-naphthalen-2-yl-ethyl, 1-naphthalen-1-yl-ethyl, 1-(4-methyl-phenyl)-ethyl, 1-(2-methoxy-phenyl)-ethyl, 1-(4-nitro-phenyl)-ethyl, indan-1-yl, or 1-(1,2,3,4-tetrahydro-naphthalen-1-yl)-ethyl, which group is of either R or S-configuration or a mixture thereof,

In another embodiment of the above methods 1, 2 or 3, where R in R1 is H, R1 is selected from benzyl, 2-methoxy benzyl, 3-methoxy benzyl, 4-methoxy benzyl, or 3,4-dimethoxy benzyl.

In another embodiment of the above methods 1, 2 or 3, R1 is benzyl, 2-methoxy benzyl, 3-methoxy benzyl, 4-methoxy benzyl, 3,4-dimethoxy benzyl, allyl, tert-butyl, diphenylmethyl or triphenylmethyl.

In a further embodiment of the compound of formula I prepared according to any of the above methods 1, 2 or 3, R1 is (S)-1-(4-methoxy-phenyl)-ethyl, yielding a compound of formula Ib and Ic.

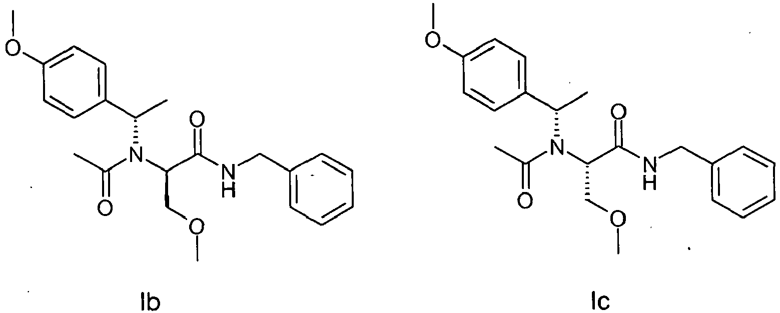

In another embodiment of the compound of formula I prepared according to any of the above methods 1, 2 or 3, R1 is (R)-1-(4-methoxy-phenyl)-ethyl, yielding a compound of formula Id and Ie.

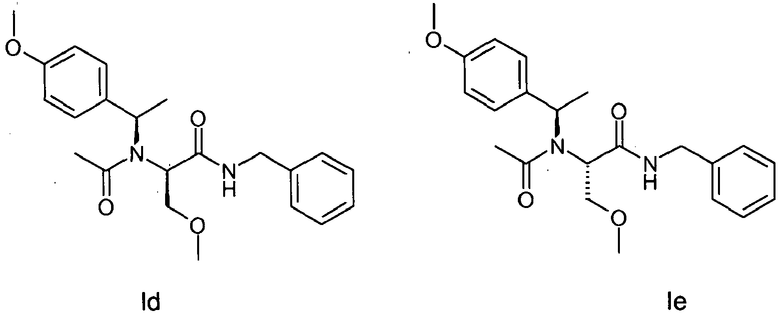

In another embodiment the compound of formula I is prepared according to any of the above methods 1, 2 or 3, by using a continuous flow process. In a continuous flow process the reaction is performed in a continuous manner by pumping the reactants either neat or dissolved in an appropriate solvent into a mixer that is maintained at a specific temperature. After a given residence time in a mixer or in a tube, the reaction is complete and the product can be isolated conventionally or using continuous isolation techniques. Several commercial and custom-made engineering solutions are available. It is of course equally possible to use a continuous-stirred-tank reactor cascade to perform the reaction in a continuous manner.

The following describes the distinct process steps in each of the methods of the invention in more detail:

According to method 1 novel compounds of formula I can be prepared by mixing acetic acid (formula VII), methoxyacetaldehyde (formula VIII), benzyl isonitrile (formula IX) and an amine of the formula X in a suitable solvent. For chiral amines of formula X, the products obtained can be formed of two possible diastereomers of formula I and la as shown in schemes 2-4. The ratio of I/Ia is ranging form 4:1 to 1:1 depending on the nature of amine X and the temperature. For achiral amines X the product I is formed as a racemate.

Solvents which can be used are alcohols such as methanol, ethanol, propanol, isopropanol, n-butanol, tert-butanol or polyethyleneglycols (PEG's). Further polar aprotic solvents like acetonitrile, tetrahydrofurane (THF), dioxane, N,N-dimethylformamide (DMF), N-methylpyrolidone (NMP) or dimethylsulfoxide (DMSO) can be used. All solvents can be used in combination with water. The water content used is ranging from 5-70%. Alcohols are preferred solvents.

The temperature used is ranging from -20°C to 100°C depending on the freezing point and the boiling point of the solvent and their mixtures, preferably at 10°C to 80°C.

In the reaction step each of the components of formula VII, VIII, IX and X can be used with one equivalent or in a slight excess for example 1.0 to 2.0 equivalents. The reaction times to obtain compounds of formula I are surprisingly short. Thus this fact allows running the reaction in a micro reactor system using a continuous flow process. Reaction times used ranging from one minute to several hours, depending, as a person skilled knows, on the nature of R1 in the amine of formula X and the reaction conditions, like solvent and temperature, as mentioned above.

The products of the formula I can be isolated by methods known in the art. For example by removal of the solvents by distillation and the residue can be purified by column chromatography on silica.

If one component was used in excess, compound I can be isolated by aqueous work up in suitable solvent, like ethylacetate (AcOEt), isopropylacetate, tert-butylmethylether (MTBE) or toluene by washing away excess acid with diluted aqueous bases, for example NaHCO₃ or KHCO₃, washing away excess base with diluted aqueous acids, for example HCl, citric acid, NaH₂PO₄ or NaHSO₄, preferably HCl, drying the organic phase for example with MgSO₄ or Na₂SO₄, and evaporation of solvents.

Further the products can be isolated by crystallisation directly from the reaction mixture or after aqueous work up depending on the nature of R1 in the reaction component X used in the reaction.

Regarding method 2 the mixture may be prepared by first mixing successively acetic acid (VII) and the amine of formula X followed by benzylisonitrile (IX) and finally adding methoxyacetaldehyde (VIII) in a suitable solvent. Solvents, reaction temperatures, equivalents are the same as described for method 1.
The order of addition described in method 2 may be beneficial. It allows running the process more safely, since the considerable reaction enthalpy from acid-base neutralisation can be removed prior the reaction heat from formation of compound of formula I will occur.

In one embodiment the amine of formula X used in the synthesis of compound I can be used as a salt such as the hydrochloride. The free amine can be released by addition of a tertiary amine like triethyl amine or diisopropylethylamine and can then be used as described in methods 1 to 3.

In one embodiment of method 3 a compound of (formula I can be prepared by simultaneous addition of a salt of formula XI - prepared by mixing acetic acid (VII) and an amine of formula X - and methoxyacetaldehyde (VIII) to benzylisonitrile (IX) in a suitable solvent.

Solvents, reaction temperatures, equivalents are the same as described for method 1. The order of addition described in method 3 may be beneficial. It prevents the chemical instable benzylisonitrile (IX) from excess of acid or bases, as described for method 1 and 2, since the isonitrile will react directly with the slowly added compounds of formula XI and VIII to a neutral compound of formula I. This results in cleaner conversions and thus in higher overall yields.

This process can be further optimised by using a continuous flow process. The advantages of continuous flow processes are well known to those skilled in the art as they are known to provide much better mixing and a much improved heat transfer. While continuous processing is generally a very useful strategy, it is most beneficial for reactions that are fast and are therefore critically depending on mixing, especially when heat has to be transferred. These criteria are met for the different embodiments of the reaction described above, so that performing the reaction by pumping the different reactants into the appropriate mixers with appropriate heating/cooling allows for a very efficient execution of the reactions described. Technically changing the order of additions is accomplished by changing the order of the pumping, piping and mixers.

The compounds of formula I as described above are obtained as mixtures of diastereomers for the case of chiral X or as racemates in case of non-chiral X (see compounds I and Ia).

For chiral X the separation into the diastereomers can be performed by any method available to those skilled in the art. A chromatographic separation of the diastereomers can be achieved using silica gel chromatography or chromatography on any other available material, such as the typical materials used for HPLC. The actual choice of chromatography conditions depends on the nature of R1. It may be economically useful to perform the chromatography using a simulated moving bed chromatography system.

Apart from chromatography, crystallizations are preferred for the separation of diastereomers. Depending on the solubility and crystalline properties of the two diastereomers I and la a separation by crystallization into the two diastereomers can be accomplished.

For non-chiral X the product I is a racemate. While numerous methods exist for the separation of enantiomers, the preferred method for the separation of I into the two enantiomers is the application of chromatography on a chiral stationary phase.
Using an appropriate chiral stationary phase with an appropriate solvent (which may be supercritical CO2 with additives) or solvent mixture a rapid and high-yielding preparation of the two enantiomers is possible. The actual choice of chromatography conditions depends on the nature of R1. It may be economically useful to perform the chromatography using a simulated moving bed chromatography system.

In another embodiment the present invention relates to the use of compound of formula I 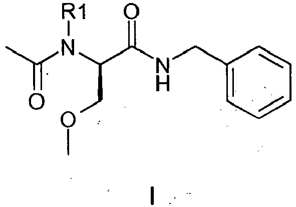 wherein R1 is defined above,
for the preparation of (R)-Lacosamide II

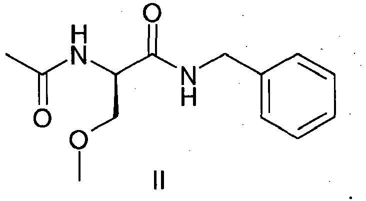

In another aspect the present invention is directed to a process for preparing (R)-Lacosamide II 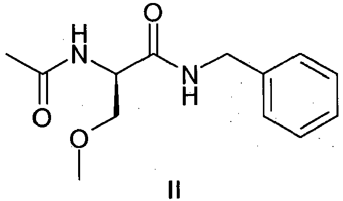 which comprises removing R1 in a compound of formula I 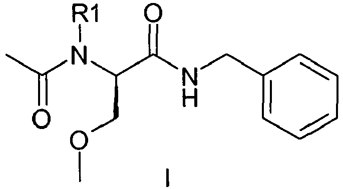 wherein R1 is as defined in a compound of formula I above,
with a suitable reagent, optionally in a suitable solvent.

In one embodiment (method 4) of this aspect the present invention is directed to the process for preparing (R)-Lacosamide (II) 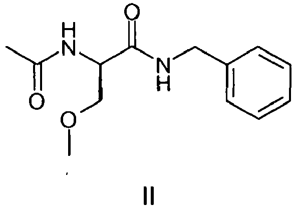 which comprises, as shown in scheme 5, 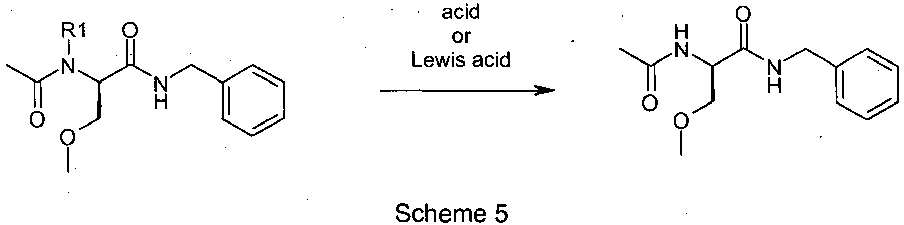 reacting a compound of the formula I
wherein
R1 is tert-butyl-, di- and triphenylmethyl-group or a
1-aryl-1-R-methyl group wherein
aryl (Ar) is a phenyl group, optionally substituted by one, two or three residues independently selected from (C₁-C₄)alkyl; (C₁-C₄)alkoxy, halogen or nitro; and
R is H or a (C₁-C₄)alkyl group with n = 1-4 carbon atoms - wherein for n = 2, 3 or 4 R can be connected to the aryl group to form a aromatic or partially unsaturated C₆-C₁₀ bicyclic residue.
with an acid or a Lewis acid.

Compound II in enantiomerically pure or enantiomerically enriched form can be obtained as described above.

The diastereomers obtained for chiral R1 can be separated by known methods such as chromatography or crystallisation. The enantiomers obtained for an achiral R1 can be separated by using chiral chromatographic techniques, but the purification techniques are not limited to these.

Acids which can be used are organic based acids such as formic acid, acetic acid, methane sulfonic acid, trifluoromethane sulfonic acid or trifluoroacetic acid, preferred are trifluoracetic acid and formic acid. Mineral acids such as HBr, HCl, HI, H₂SO₄ or H₃PO₄ can also be used. The acids may be used alone or as a mixture of at least two of the aforesaid acids.

These acids can be used neat or in combination with solvents. Solvents which can be used in this step are non polar such as dichloromethane, toluene, or ether type solvents such as THF, dioxane, anisole or MTBE, or protic solvents such as water or alcohols. Furthermore, Lewis acids like boron trifluoride, titanium tetrachloride, alumina trichloride, ferric chloride or zinc chloride in combination with aprotic solvents like dichloromethane, toluene, or ether type solvents such as THF, dioxane, anisole or MTBE can be used in this step.

The temperature used is ranging from 0 °C to 100 °C depending on the boiling point of the solvent, the kind of acid used as well as the nature of R1 in the compounds of formula I.
(R)-Lacosamide (formula II) can be isolated in a way known by a person skilled in the art. For example by removal of the solvents and the residue can be purified by column chromatography on silica or by crystallisation.
(R)-Lacosamide (formula II) can be further isolated by aqueous work up in suitable solvent, like ethylacetate (AcOEt), isopropylacetate, dichloromethane or toluene by washing away excess acid with diluted aqueous bases, for example NaHCO₃ or KHCO₃ washing away excess base with diluted aqueous acids, for example HCl, citric acid, drying the organic phase for example with MgSO₄ or Na₂SO₄, and evaporation of solvents.

In a special embodiment when formic acid is used as acid, toluene, ethylbenzene, xylene, benzene or ether-type solvents can be added and the impurities can be removed by liquid/liquid biphasic extraction with the biphasic system for instance formic acid/toluene. The product (R)-Lacosamide (formula II) can be collected after concentration of the formic acid layer using techniques well known to those skilled in the art.

In a preferred embodiment (R)-Lacosamide (formula II) is prepared according to method 4, by treatment of a compound of formula Ib wherein R1 is (S)-1-(4-methoxy-phenyl)-ethyl as shown in scheme 6.

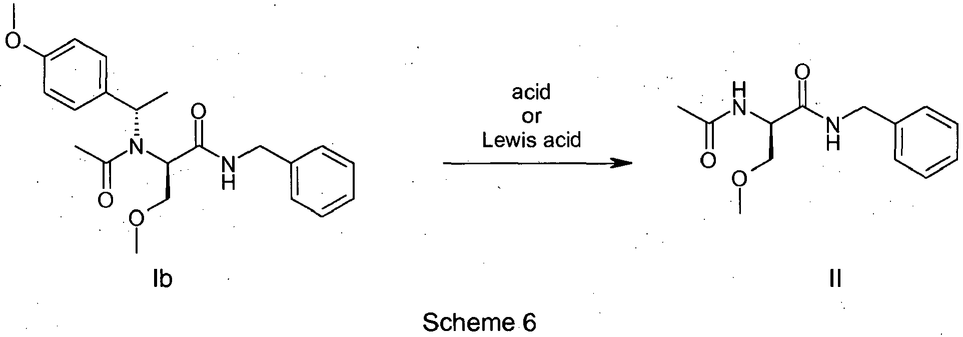

In another preferred embodiment (R)-Lacosamide (formula II) is prepared according to method 4, by treatment of a compound of formula Id wherein R1 is (R)-1-(4-methoxy-phenyl)-ethyl as shown in scheme 7.

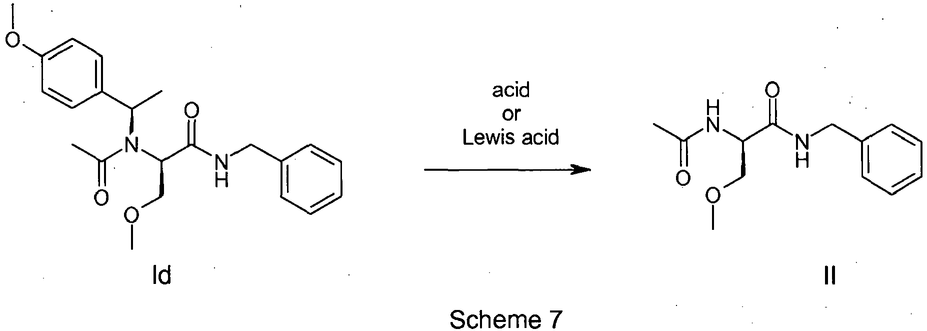

In a further embodiment (method 5) (R)-Lacosamide can be obtained by treatment of a compound of formula I with a hydrogen source and a transition metal in a suitable solvent. Thus the present invention also relates to a process for preparing (R)-Lacosamide (formula II) 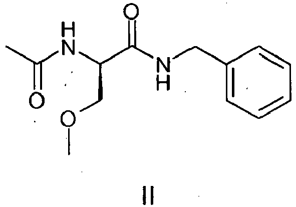 which comprises, as shown in scheme 8, 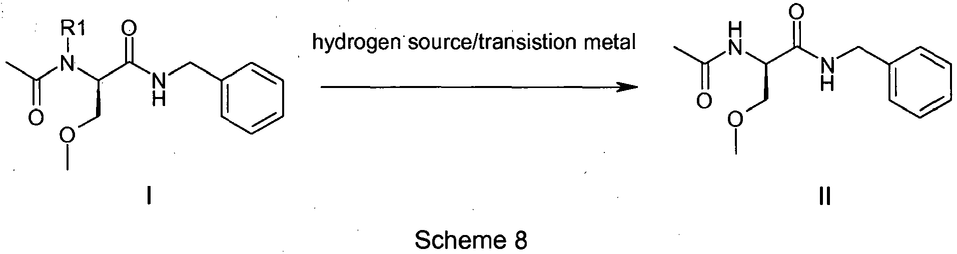 reacting a compound of the formula I, wherein
R1 is diphenylmethyl, triphenylmethyl or
a 1-aryl-1-R-methyl group wherein
aryl (Ar) is a phenyl group, optionally substituted by one, two or three residues independently selected from (C₁-C₄)alkyl; (C₁-C₄)alkoxy, halogen or nitro; and
R is H or a (C₁-C₄)alkyl group with n = 1-4 carbon atoms - wherein for n = 2, 3 or 4 R can be connected to the aryl group to form a aromatic or partially
unsaturated C₆-C₁₀ bicyclic residue.
with a hydrogen source in the presence of a transition metal.

In one embodiment of the above methods 4 or 5, where R in R1 is (C₁₋C₄)alkyl, R1 is selected from 1-(4-methoxy-phenyl)-ethyl, 1-phenyl-ethyl, 1-phenyl-propyl, 1-naphthalen-2-yl-ethyl, 1-naphthalen-1-yl-ethyl, 1-(4-methyl-phenyl)-ethyl, 1-(2-methoxy-phenyl)-ethyl, 1-(4-nitro-phenyl)-ethyl, indan-1-yl or 1-(1,2,3,4-tetrahydronaphthalen-1-yl)-ethyl, which group is of either R or S-configuration or a mixture thereof,

In another embodiment of the above methods 4 or 5, where R in R1 is H, R1 is selected from benzyl, 2-methoxy benzyl, 3-methoxy benzyl, 4-methoxy benzyl, or 3,4-dimethoxy benzyl.

Hydrogen equivalents which can be used are hydrogen, formic acid and their salts, such as ammonium formate or cyclohexadiene, preferred is hydrogen.
Transition metals which can be used are Palladium, Rhodium, Platinum or Iridium, preferred is Palladium.

Acids as described above in connection with the acid cleavage may be added in the hydrogenation reaction to facilitate cleavage. In particular the reaction can be carried out in presence of acetic acid, 2-chloro- acetic acid, methoxy-acetic acid, propionic acid, butyric acid, valeric acid or pivalic acid or a mixture of at least 2 of aforesaid acids, preferably acetic acid is used.

(R)-Lacosamide (formula II) can be isolated in a way known by a person skilled in the art. For example by removal of the catalyst by filtration, followed by removal of the solvents and the residue can be purified by column chromatography on silica or by crystallisation.

In a further embodiment (method 6) of this aspect the present invention relates to a process for preparing (R)-Lacosamide (II) 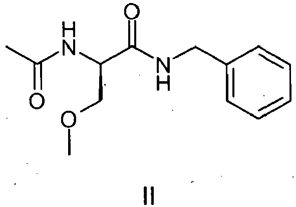 which comprises, as shown in scheme 9, 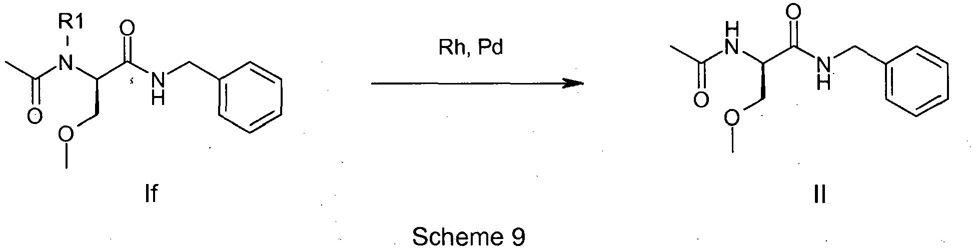 reacting a compound of formula If, wherein R1 is allyl, with a transition metal. Suitable transitions metals for use in this process are Rhodium or Palladium. The removal of the allyl group is described in T. W. Greene and P. G. M. Wuts: Protective Groups in Organic Synthesis, Third Edition, John Wiley and Sons, New York, 1999, 574-576.

### Abbreviations:

- Ac: acetyl
- bs: broad singulett
- ca.: circa
- d: day, dublett
- dr: diasteromeric ratio
- de: diastereromic excess
- DBU: 1,8-diaza-bicyclo[5.4.0]-undec-7-ene
- Eq.: equivalents
- EtOH: ethanol
- h: hour(s)
- i.vac.: in vacuum
- LC-MS: liquid chromatography-mass spectrometry
- m: multiplett
- M: molar, concentration (mol/liter)
- Me: methyl
- MeOH: methanol
- MIBK: methyl-isobutyl ketone
- Min: minutes
- MTBE: methyl-tert.-butylether
- NMR: Nuclear magnetic resonance
- q: quartett
- rt: room temperature
- RP-HPLC: reversed phase high performance liquid chromatography
- s: singulett
- TFA: trifluoracetic acid
- THF: tetrahydrofuran

### Examples

This invention is described in more detail by the examples that follow. These examples are designated to illustrate the invention, but do not limit its scope. Each step of the process described in the present invention may be operated either batch by batch or as a continuous process, or semicontinuous mode, and is scalable on larger amounts than described here.

The NMR assignments are for illustration only based on analysis of the one-dimensional ¹H NMR spectra as done by those skilled in the art. A more detailed analysis of the spectra may lead to minor reassignments of some NMR peaks, which obviously does not change the overall assignment. All ¹H NMR spectra are recorded on a 500 MHz instrument at rt or on a 250 MHz instrument at 390 K (rotamers). At 390 K some of the NH-protons disappeared by a broad signal. Shifts are relative to TMS in [ppm]; the solvent is always DMSO-d₆.

General procedure 1 for preparing compounds of formula I according to method 1: The appropriate amine X was added to a mixture of methoxyacetaldehyde, Acetic acid and benzylisonitrile in MeOH and water. The mixture was stirred for the appropriate time at rt. MTBE was added, the phases were separated and the organic layer was washed successively with aq. NaHSO₄, sat. sodium bicarbonate, brine, dried with MgSO₄ and concentrated.

General procedure 2 for preparing compounds of formula I according to method 2: The appropriate amine X was dissolved in MeOH. Acetic acid was added, followed by benzylisonitrile. Methoxyacetaldehyde, in water, was slowly added. The mixture was stirred for the appropriate time at rt. MTBE was added, the phases were separated and the organic layer was washed successively with aq. NaHSO₄, sat. sodium bicarbonate, brine, dried with MgSO₄ and concentrated.

General procedure 3 for preparing compounds of formula I according to method 3: A salt of formula XI was prepared by mixing an appropriate amine X and acetic acid in MeOH and water. Benzylisonitrile was dissolved in MeOH and heated to 65°C. To this mixture the solution of salt XI and aqueous methoxyacetaldehyde solution were simultaneously added. The mixture was stirred for the appropriate time at 65°C. The product was obtained after aqueous work up as described in general procedure 1 and 2, or by direct crystallisation from the reaction mixture.

### Example 1: (R)-2-{Acetyl-[(R)-1-(4-methoxy-phenyl)-ethyl]-amino}-N-benzyl-3-methoxy-propionamide Id (a compound of the formula I, R1 = (R)-1-(4-methoxy-phenyl)-ethyl)

1.84 g (12.2 mmol) of (R)-1-(4-Methoxyphenyl)ethylamine (a compound of the formula X, R1 = (R)-1-(4-methoxy-phenyl)-ethyl), 0.73 g (12.2 mmol) acetic acid, 1.27 g (10.8 mmol) Benzylisonitrile and 1 g (13.5 mmol) methoxyacetaldehyde in 12 ml MeOH/water 1:1, were allowed to react for 30 min as described in general procedure 1 to yield 3.97 g (95%) of crude title compound Id as a 35/65 diastereomeric mixture (Id/Ie). An aliquot of this sample was purified by advanced preparative chromatography to gave pure minor (R,R)-diastereomer Id as a colourless solid. Chiral HPLC (Chiralpak AD-H/120 (250x4.6 mm), Heptane:EtOH:MeOH 2:1:1 +0.1% TFA, 30°C, 1 ml/min): Rₜ = 6.62 min; HPLC (Merck Chromolith Performance RP18e, A. H₂O/0.05% TFA, B: MeCN/0.05% TFA, 10->70% B 10 min, 4 ml/min, 40°C): Rₜ = 3.51 min; NMR (250 MHz, 390 K): 1.58 (d, 3H, CH₃), 2.07 (s, 3H, Ac), 3.32 (s, 3H, OMe), 3.71 (dd, 1 H, CH₂-OMe), 3.75 (s, 3H, OMe), 3.96 (dd, 2H, CH₂-OMe), 4.07 -4.25 (m, 3H, CH, CH₂Ph), 5.23 (q, 1 H, CH), 6.777-6.85 (m, 2H, Ar-H), 7.06-7.13 (m, 2H, Ar-H), 7.20-7.36 (m, 5H, Ar-H); LC-MS: MH⁺ 385 (250, 135).
Major (R,S)-diastereomer Ie was obtained after advanced prep. chromatography as an oil. Chiral HPLC (vide supra): Rₜ = 4.86 min; HPLC (vide supra): Rₜ = 3.65 min; LC-MS : MH⁺ 385 (250, 135).

### Example 2: (R)-2-{Acetyl-[(S)-1-(4-methoxy-phenyl)-ethyl]-amino}-N-benzyl-3-methoxy-propionamide Ib (a compound of the formula I, R1 = (S)-1-(4-methoxyphenyl)-ethyl)

4.49 g (29.7 mmol) of (S)-1-(4-Methoxyphenyl)ethylamine (a compound of the formula X, R1 = (S)-1-(4-methoxy-phenyl)-ethyl), 1.89 g (31.5 mmol) acetic acid, 3.28 g (28 mmol) benzylisonitrile and 2.6 g (35 mmol) methoxyacetaldehyde in 10 ml water, were allowed to react in 10 ml MeOH for 30 min as described in general procedure 2 to yield 11 g (quant. yield) of crude title compound Ib as a 65/35 diastereomeric mixture (Ib/Ic).

An aliquot of this sample was purified by advanced preparative chromatography to gave pure major (S,R)-diastereomer Ib as a yellow oil: Chiral HPLC (Chiralpak AD-H/120 (250x4.6 mm), Heptane:EtOH:MeOH 2:1:1 +0.1% TFA, 30°C, 1 ml/min): Rₜ = 5.96 min; HPLC (Merck Chromolith Performance RP18e, A. H₂O/0.05% TFA, B: MeCN/0.05% TFA, 10->70% B 10 min, 4 ml/min, 40°C): Rₜ = 3.65 min; NMR (250 MHz, 390 K): 1.55 (d, 3H, CH₃), 2.07 (s, 3H, Ac), 3.03 (s, 3H, OMe), 3.28 (dd, 1 H, CH or CH₂-OMe), 3.79 (s, 3H, OMe), 3.92-4.10 (m, 2H, CH or CH₂-OMe), 4.28 -4.46 (m, 2H, CH₂Ph), 5.18 (q, 1 H, ArCH), 6.88-6.96 (m, 2H, Ar-H), 7.20-7.38 (m, 7H, Ar-H), 7.71 (bs, 1 H, NH);.LC-MS: MH⁺ 385 (250, 135).

Minor (S,S)-diastereomer Ic was obtained after advanced prep. chromatography as a colourless solid. Chiral HPLC (vide supra): Rt = 4.19 min; HPLC (vide supra): Rₜ = 3.51 min; NMR (250 MHz, 390 K): 1.58 (d, 3H, CH₃), 2.07 (s, 3H, Ac), 3.32 (s, 3H, OMe), 3.68-3.78 (m, 4H, CH or CH₂-OMe, OMe), 3.97 (dd, 1 H, CH or CH₂-OMe), 4.08 -4.22 (m, 3H, CH or CH₂-OMe, CH₂Ph), 5.24 (q, 1 H, ArCH), 6.76-6.86 (m, 2H, Ar-H), 7.07-7.37 (m, 7H, Ar-H); LC-MS: MH⁺ 385 (250, 135).

This residue diastereomeric mixture (10.8 g) was dissolved in 90 ml MTBE with heating. After cooling to 40°C seed crystals of the minor (S,S)-diastereomer Ic were added and the mixture was stirred for 4 d. The mixture was cooled to 0°C, filtrated and washed with cold MTBE to yield about 3 g of the minor (S,S)-Ic with 98:2 dr. The mother liquor was concentrated to yield about 8 g of the title compound. HPLC (vide supra) revealed that the content of the (S,R)-diastereomer Ib was enriched from 65:35 to 88:12.

### Example 3: (R)-2-[Acetyl-((S)-1-phenyl-ethyl)-amino]-N-benzyl-3-methoxy-propionamide (a compound of the formula I, R1 = (S)-1-phenyl)-ethyl)

0.5 g (4.1 mmol) of (S)-1-(Phenyl)ethylamine (a compound of the formula X, R1 = (S)-1-(phenyl)-ethyl), 0.5 g (4.1 mmol) acetic acid, 0.39 g (3.3 mmol) benzylisonitrile and 4.1 mmol methoxyacetaldehyde, were allowed to react in in 13 ml acetonitrile/MeOH/water 8:4:1 for 30 min as described in general procedure 1 to yield 0.43 g (37%) of the title compound as a 60/40 diastereomeric mixture after column chromatography on 40 g silica with heptane/AcOEt 1:1 -> AcOEt. An aliquot of this sample was purified by advanced preparative chromatography to gave the major (S,R)-diastereomer as a yellow oil. Chiral HPLC (Chiralpak AD-H/83 (250x4.6 mm), EtOH, 30°C, 0.75 ml/min): Rₜ = 8.18 min; HPLC (Merck Chromolith Performance RP18e, A. H₂O/0.05% TFA, B: MeCN/0.05% TFA, 10->70% B 10 min, 4 ml/min, 40°C): Rₜ = 3.67 min; NMR (250 MHz, 390 K): 1.59 (d, 3H, CH₃), 2.07 (s, 3H, Ac), 3.02 (s, 3H, OMe), 3.29 (dd, 1 H, CH or CH₂-OMe), 3.94 (dd, 1 H, CH or ArCH), 7.19-7.43 (m, 10H, Ar-H), 7.73 (bs, 1 H, NH); LC-MS: MH⁺ 355.
Minor (S,S)-diastereomer was obtained after advanced prep. chrom. as a yellow oil Chiral HPLC (vide supra): Rₜ = 5.73 min; HPLC (vide supra): Rₜ = 3.53 min; NMR (250 MHz, 390 K): 1.62 (d, 3H, CH₃), 2.06 (s, 3H, Ac), 3.32 (s, 3H, OMe), 3.73 (dd, 1 H, CH or CH₂-OMe), 3.94 (dd, 1 H, CH or CH₂-OMe), 4.12-4.25 (m, 3H, CH, CH₂Ph), 5.27 (q, 1H, CH), 7.07=7.44 (m, 10H, Ar-H); LC-MS: MH⁺ 355.

### Example 4: (R)-2-[Acetyl-((S)-1-naphthalen-1-yl-ethyl)-amino]-N-benzyl-3-methoxy-propionamide (a compound of the formula I, R1 = (S)-1-(naphthalen-1-yl)-ethyl)

0.58 g (3.4 mmol) of (S)-1-(Naphthalen-1-yl)-ethylamine (a compound of the formula X, R1 = (S)-1-(naphthalen-1-yl)-ethyl), 0.22 g (3.7 mmol) acetic acid, 0.39 g (3.3 mmol) benzylisonitrile and 2.5 ml (4.3 mmol) methoxyacetaldehyde (1.7 M in water), were allowed to react in 3 ml MeOH for 18 h as described in general procedure 2 to yield 1.3 g (quant. yield) of crude title compound as a 60/40 diastereomeric mixture. An aliquot of this sample was purified by advanced preparative chromatography to gave pure major (S,R)-diastereomer as a colourless oil. Chiral HPLC (Chiralpak AD-H/120 (250x4.6 mm), EtOH, 30°C, 1 ml/min): Rₜ = 7.17 min; HPLC (Merck Chromolith Performance RP18e, A. H₂O/0.05% TFA, B: MeCN/0.05% TFA, 10->70% B 10 min, 4 ml/min, 40°C): Rₜ = 4.46 min; NMR (250 MHz, 390 K): 1.67 (d, 3H, CH₃), 2.13 (s, 3H, Ac), 2.52 (s, 3H, OMe), 2.55 (dd, 1 H, CH or CH₂-OMe), 3.71 (dd, 1 H, CH or CH₂-OMe), 3.85 (dd, 1 H, CH or CH₂-OMe), 4.26 -4.44 (m, 2H, CH₂Ph), 6.18 (q, 1 H, ArCH), 7.18-8.02 (m, 13H, Ar-H, NH); LC-MS: MH⁺ 405.
Minor (S,S)-diastereomer was obtained after advanced prep. chrom. as a colourless solid. Chiral HPLC (vide supra): Rₜ = 4.98 min; HPLC (vide supra): Rₜ = 4.32 min; NMR (250 MHz, 390 K): 1.70 (d, 3H, CH₃), 2.18 (s, 3H, Ac), 3.32 (s, 3H, OMe), 3.55-3.78 (m, 3H, CH₂Ph, CH or CH₂-OMe), 3.88 (dd, 1 H , CH or CH₂-OMe), 4.00 (dd, 1 H , CH or CH₂-OMe), 6.11 (bs, 1 H, NH), 6.25 (q, 1 H, ArCH), 6.64-6.73 (m, 2H, Ar-H), 7.12-7.20 (m, 3H, Ar-H), 7.41-7.55 (m, 3H, Ar-H), 7.70 (d, 1H, Ar-H), 7.83-7.94 (m, 2H, Ar-H), 8.01-8.10 (m, 1H, Ar-H); LC-MS: MH⁺ 405.

### Example 5: (R)-2-[Acetyl-((S)-1-naphthalen-2-yl-ethyl)-amino]-N-benzyl-3-methoxy-propionamide (a compound of the formula I, R1 = (S)-1-(naphthalen-2-yl)-ethyl)

15.4 g (90.0 mmol) of (S)-1-(Naphthalen-2-yl)-ethylamine (a compound of the formula X, R1 = (S)-1-(naphthalen-2-yl)-ethyl), 5.9 g (99 mmol) acetic acid, 10.2 g (87.0 mmol) benzylisonitrile and 53 ml (90 mmol) methoxyacetaldehyde (1.7 M in water), were allowed to react in 100 ml MeOH for 14 h as described in general procedure 2 to yield 36.5 g (quant. yield) of crude title compound of as a 60/40 diastereomeric mixture. An aliquot of this sample was purified by advanced preparative chromatography to gave pure major (S,R)-diastereomer as a colourless solid. Chiral HPLC (Chiralpak AD-H/91 (250x4.6 mm), EtOH:MeOH 1:1, 30°C, 1 ml/min): Rₜ = 9.82 min; HPLC (Merck Chromolith Performance RP18e, A. H₂O/0.05% TFA, B: MeCN/0.05% TFA, 10->70% B 10 min, 4 ml/min, 40°C): Rₜ = 4.50 min; NMR (250 MHz, 390 K): 1.70 (d, 3H, CH₃), 2.12 (s, 3H, Ac), 2.93 (s, 3H, OMe), 3.35 (dd, 1 H, CH or CH₂-OMe), 3.95 (dd, 1 H, CH or CH₂-OMe), 4.13 -4.47 (m, 3H, CH or CH₂-OMe, CH₂Ph), 5.37 (q, 1H, ArCH), 7.19-7.93 (m, 13H, Ar-H, NH); LC-MS: MH⁺ 405. Minor (S,S)-diastereomer was obtained after advanced prep. chromatography as a colourless solid. Chiral HPLC (vide supra): Rₜ = 4.46 min; HPLC (vide supra): Rₜ = 4.34 min; NMR (250 MHz, 390 K): 1.73 (d, 3H, CH₃), 2.12 (s, 3H, Ac), 2.83 (s, 3H, OMe), 3.78 (dd, 1 H, CH or CH₂-OMe), 3.97 (dd, 1 H, CH or CH₂-OMe), 4.02 -4.29 (m, 3H, CH or CH₂-OMe, CH₂Ph), 5.44 (q, 1 H, ArCH), 6.94-7.92 (m, 13H, Ar-H, NH); LC-MS: MH⁺ 405.

This residue diastereomeric mixture (about 30 g) was dissolved in 200 ml MTBE with heating. After cooling to 35°C seed crystals of the minor (S,S)-diastereomer were added and the mixture was stirred for 48 h. The mixture was cooled to 0°C, filtrated and washed with cold MTBE to yield 5.2 g of the minor (S,S)-diastereomer with 98 % de. The mother liquor was concentrated to yield about 25 g of the title compound as a viscuous brown oil. HPLC (vide supra) revealed that the content of the (S,R)-diastereomer was enriched from 60:40 to 70:25. The residue mother liquor was dissolved in 100 ml heptane/AcOEt 1:1 with heating. After cooling to 35°C seed crystals of the major (S,R)-diastereomer were added and the mixture was stirred for 24 h. The mixture was cooled to 0°C, filtrated and washed with cold heptane/AcOEt to yield 13.4 g (37%) of the title compound as a colourless solid (98:2 dr).

### Example 6: (R)-2-{Acetyl-[(R)-1-(2-methoxy-phenyl)-ethyl]-amino}-N-benzyl-3-methoxy-propionamide (a compound of the formula I, R1 = (R)-1-(2-methoxy-phenyl)-ethyl)

1.0 g (6.6 mmol) of (R)-1-(2-Methoxyphenyl)ethylamine (a compound of the formula X, R1 = (R)-1-(2-methoxy-phenyl)-ethyl), 0.40 g (6.6 mmol) acetic acid, 0.74 g (6.3 mmol) benzylisonitrile and 4.8 ml (8.3 mmol) methoxyacetaldehyde (1.6 M in water), were allowed to react in 5 ml MeOH for 18 h as described in general procedure 2 to yield 2.3 g (97%) of crude title compound of as a 40/60 diastereomeric mixture. An aliquot of this sample was purified by advanced preparative chromatography to gave pure minor (R,R)-diastereomer as colourless solid. Chiral HPLC (Chiralpak AD-H/83 (250x4.6 mm), Heptane:EtOH 5:1 +0.1% TFA, 30°C, 1 ml/min): Rₜ = 17.1 min; HPLC (Merck Chromolith Performance RP18e, A. H₂O/0.05% TFA, B: MeCN/0.05% TFA, 10->70% B 10 min, 4 ml/min, 40°C): Rₜ = 3.80 min; NMR (500 MHz, 300 K): 1.50 (d, H, CH₃), 2.25 (s, 3H, Ac), 3.26 (s, 3H, OMe), 3.33 (1 H, covered by H₂O-signal), 3.51 (s, 3H, OMe), 3.61 (dd, 1 H, CH or CH₂-OMe), 3.98 (dd, 1 H, CH or CH₂-OMe), 4.02-4.13 (m, 3H, CH₂Ph, CH or CH₂-OMe) 5.35 (q, 1 H, ArCH), 6.45 (t, 1 H, NH), 6.87 (d, 1 H, Ar-H), 6.99 (d, 2H, Ar-H), 7.08 (dd, 1 H, Ar-H), 7.22-7.46 (m, 5H, Ar-H); LC-MS: MH⁺ 385 (250, 135).

Major (R,S)-diastereomer was obtained after advanced prep. chrom. as a yellow oil. Chiral HPLC (vide supra): Rₜ - 9.77 min; HPLC (vide supra): Rₜ = 3.83 min; NMR (500 MHz, 300 K): 1.51 (d, 3H, CH₃), 2.19 (s, 3H, Ac), 2.70 (s, 3H, OMe), 3.33 (1 H, covered by H₂O-signal), 3.51 (dd, 1 H, CH or CH₂-OMe), 3.84 (s, 3H, OMe), 4.11 (dd, 1 H, CH or CH2-OMe), 4.28 (dd, 1 H, CH₂-Ph), 4.39 (dd, 1 H, CH₂-Ph), 5.33 (q, 1 H, ArCH), 7.01 (dd, 1 H, Ar-H), 7.07 (d, 1 H, Ar-H), 7.22-7.49 (m, 7H, Ar-H), 7.60 (t, 1 H, NH)LC-MS : MH⁺ 385 (250, 135).

### Example 7: (R)-2-{Acetyl-[(S)-1-(4-nitro-phenyl)-ethyl]-amino}-N-benzyl-3-methoxy-propionamide (a compound of the formula I, R1 = (S)-1-(4-nitro-phenyl)-ethyl)

1.4 g (6.9 mmol) of (S)-1-(4-Nitrophenyl)ethylamine hydrochlorid (a compound of the formula X, R1 = (S)-1-(4-nitro-phenyl)-ethyl) in 5 ml MeOH was treated with 0.70 g (6.9 mmol) triethylamine at rt. After stirring for 10 min 0.44 g (7.2 mmol) acetic acid, 0.74 g (6.3 mmol) benzylisonitrile and 4.7 ml (7.6) mmol methoxyacetaldehyde (1.6 M in water) were allowed to for 18 h as described in general procedure 2 to yield 2.5 g (quant. yield) of crude title compound as a 65/35 diastereomeric mixture. An aliquot of this sample was purified by advanced preparative chromatography to gave pure major (S,R)-diastereomer as a yellow oil. Chiral HPLC (Chiralpak AD-H/120 (250x4.6 mm), EtOH, 30°C, 1 ml/min): Rₜ = 9.44 min; HPLC (Merck Chromolith Performance RP18e, A. H₂O/0.05% TFA, B: MeCN/0.05% TFA, 10->70% B 10 min, 4 ml/min, 40°C): Rₜ = 3.61 min; NMR (250 MHz, 390 K): 1.65 (d, 3H, CH₃), 2.08 (s, 3H, Ac), 3.13 (s, 3H, OMe), 3.52 (dd, 1H, CH₂-OMe), 3.85 (dd, 1H, CH2-OMe), 4.27-4.45 (m, 3H, CH, CH₂Ph), 5.19 (q, 1H, ArCH), 7.19-7.37 (m, 5H, Ar-H), 7.59-7.68 (m, 2H, Ar-H), 7.99 (bs, 1 H, NH), 8.09-8.18 (m, 2H, Ar-H); LC-MS: MH⁺ 400.

Minor (S,S)-diastereomer was obtained after advanced prep. chrom. as a yellow oil. Chiral HPLC (vide supra): Rₜ = 5.26 min; HPLC (vide supra): Rₜ = 3.50 min ; NMR (250 MHz, 390 K): 1.68 (d, 3H, CH₃), 2.08 (s, 3H, Ac), 3.35 (s, 3H, OMe), 3.74-3.92 (m, 2H, CH₂-OMe), 4.13-4.30 (m, 2H, CH₂Ph), 4.43 (dd, 1 H, CH), 5.22 (q, 1 H, ArCH), 7.12-7.30 (m, 5H, Ar-H), 7.54-7.62 (m, 2H, Ar-H), 7.97-8.04 (m, 2H, Ar-H); LC-MS: MH⁺ 400.

### Example 8: (R)-2-[Acetyl-((S)-1-phenyl-propyl)-amino]-N-benzyl-3-methoxy-propionamide (a compound of the formula I, R1 = (S)-1-(phenyl)-propyl)

0.85 g (6.3 mmol) of (S)-1-(Phenyl)propylamine (a compound of the formula X, R1 = (S)-1-(phenyl)-propyl), 0.44 g (7.2 mmol) acetic acid, 0.74 g (6.3 mmol) benzylisonitrile and 4.0 ml (7.6 mmol) methoxyacetaldehyde (1.9 M in water), were allowed to react in 4 ml MeOH for 90 min as described in general procedure 2. The mixture was concentrated and distilled three times with 10 ml toluene to yield 2.3 g (99%) of the title compound as a 65/35 diastereomeric mixture. An aliquot of this sample was purified by advanced preparative chromatography to gave the pure major (S,R)-diastereomer as a yellow oil: Chiral HPLC (Chiralcel OJ-H/77 (250x4.6 mm), heptane:EtOH 5:1, 30°C, 1.0 ml/min): Rₜ = 9.20 min; HPLC (Merck Chromolith Performance RP18e, A. H₂O/0.05% TFA, B: MeCN/0.05% TFA, 10->70% B 10 min, 4 ml/min, 40°C): Rₜ = 4.04 min; NMR (250 MHz, 390 K): 0.92 (t, 3H, CH₃), 1.96-2.12 (m, 2H, CH₂), 2.15 (s, 3H, Ac), 2.92 (s, 3H, OMe), 2.93-3.00 (m, 1 H, CH or CH₂-OMe), 3.87-4.00 (m, 2H, CH or CH₂₋OMe), 4.26-4.44 (m, 2H, CH₂Ph), 4.97 (t, 1 H, ArCH), 7.17-7.47 (m, 10H, Ar-H), 7.72 (bs, 1 H, NH); LC-MS: MH⁺ 369.
Minor (S,S)-diastereomer was obtained after advanced prep. chrom. as a yellow oil: Chiral HPLC (vide supra): Rₜ = 5.48 min; HPLC (vide supra): Rₜ = 3.90 min ; NMR (250 MHz, 390 K): 0.94 (t, 3H, CH₃), 1.88-2.25 (m, 2H, CH₂), 2.15 (s, 3H, Ac), 3.32 (s, 3H, OMe), 3.66-3.77 (m, 1 H, CH or CH₂-OMe), 3.92-4.13 (m, 4H, CH or CH₂-OMe, CH₂Ph), 5.02 (t, 1 H, ArCH), ), 6.82 (bs, 1 H, NH), 6.97-7.06 (m, 2H, Ar-H), 7.17-7.32 (m, 6H, Ar-H), 7.38-7.46 (m, 2H, Ar-H); LC-MS: MH⁺ 369.

### Example 9: (R)-2-((S)-Acetyl-indan-1-yl-amino)-N-benzyl-3-methoxy-propionamide (a compound of the formula I, R1 = (S)-indan-1-yl)

0.84 g (6.3 mmol) of (S)-Indan-1-ylamine (a compound of the formula X, R1 = (S)-indan-1-yl), 0.44 g (7.2 mmol) acetic acid, 0.74 g (6.3 mmol) benzylisonitrile and 4.0 ml (7.6 mmol) methoxyacetaldehyde (1.9 M in water), were allowed to react in 4 ml MeOH for 18 h as described in general procedure 2. The mixture was concentrated and distilled three times with 10 ml toluene to yield 2.3 g (100%) of the title compound as a 55/45 diastereomeric ratio. An aliquot of this sample was purified by advanced preparative chromatography to gave the pure major (S,R)-diastereomer as a yellow oil: Chiral HPLC (Chiralpak AD-H/120 (250x4.6 mm), EtOH, 30°C, 1.0 ml/min): Rₜ = 6.85 min; HPLC (Merck Chromolith Performance RP18e, A. H₂O/0.05% TFA, B: MeCN/0.05% TFA, 10->70% B 10 min, 4 ml/min, 40°C): Rₜ = 3.88 min; NMR (250 MHz, 390 K): 1.98 (s, 3H, Ac), 2.11-2.43 /m, 2H, CH₂), 2.70-3.06 (m, 2H, CH₂Ar), 3.19 (s, 3H, OMe), 3.66 (dd, 1H, CH or CH₂-OMe), 4.19 (dd, 1H, CH or CH₂-OMe), 4.21-4.44 (m, 3H, CH₂Ph, CH or CH₂-OMe), 5.34 (dd, 1 H, ArCH), 7.15-7.37 (m, 9H, Ar-H), 7.82 (bs, 1 H, NH); LC-MS: MH⁺ 369.
Major (S,S)-diastereomer was obtained after advanced prep. chromatography as a yellow oil: Chiral HPLC (vide supra): Rₜ = 5.51 min, HPLC (vide supra): Rₜ = 3.81 min ; NMR (250 MHz, 390 K): 1.94 (s, 3H, Ac), 2.07-2.24 (m, 1 H, CH₂), 2.34-2.47 (m, 1 H, CH₂), 2.75-2.90 (m, 1 H, CH₂Ar), 2.97-3.11 (m, 1 H, CH₂Ar), 3.29 (s, 3H, OMe), 3.74 (dd, 1 H, CH or CH₂-OMe), 3.90 (dd, 1 H, CH or CH₂-OMe), 4.20-4.41 (m, 3H, CH₂Ph, CH or CH₂-OMe), 5.41 (dd, 1 H, ArCH), 6.99-7.36 (m, 9H, Ar-H), 7.87 (bs, 1 H, NH); LC-MS: MH⁺ 369.

### Example 10: (R)-2-((S)-Acetyl-1,2,3,4-tetrahydro-naphthalen-1-yl-amino)-N-benzyl-3-methoxy-propionamide (a compound of the formula I, R1 = (S)-1,2,3,4-tetrahydro-naphthalen-1-yl)

0.93 g (6.3 mmol) of (S)-1,2,3,4-tetrahydro-naphthalen-1-ylamine (a compound of the formula X, R1 = (S)-1,2,3,4-tetrahydro-naphthalen-1-yl)), 0.44 g (7.2 mmol) acetic acid, 0.74 g (6.3 mmol) benzylisonitrile and 4.0 ml (7.6 mmol) methoxyacetaldehyde (1.9 M in water), were allowed to react in 4 ml MeOH for 18 h as described in general procedure 2. The mixture was concentrated and distilled three times with 10 ml toluene to yield 2.4 g (100%) of the title compound as a 50/50 diastereomeric ratio. An aliquot of this sample was purified by advanced preparative chromatography to gave the pure (S,R)-diastereomer) as a colorless oil: Chiral HPLC (Chiralpak AD-H/120 (250x4.6 mm), heptane:EtOH 1:1, 30°C, 1.0 ml/min) Rₜ = 8.33 min; HPLC (Merck Chromolith Performance RP18e, A. H₂O/0.05% TFA, B: MeCN/0.05% TFA, 10->70% B 10 min, 4 ml/min, 40°C): Rₜ = 4.14 min; NMR (250 MHz, 390 K): 1.54-2.08 (m, 4H, CH₂), 2.00 (s, 3H, OAc), 2.61-2.87 (m, 2H, CH₂Ar), 3.16 (s, 3H, OMe), 3.65 (dd, 1 H, CH or CH₂-OMe), 3.98 (dd, 1 H, CH or CH₂-OMe), 4.20-4.42 (m, 3H, CH₂Ph, CH or CH₂-OMe), 5.02 (dd, 1 H, ArCH), 7.04-7.37 (m, 9H, Ar-H), 7.85 (bs, 1 H, NH); LC-MS: MH⁺ 381.
(S,S)-diastereomer was obtained after advanced prep. chrom. (vide supra) as a colorless oil: Rₜ = 6.05 min, HPLC (vide supra) : Rₜ = 4.07 min ; NMR (250 MHz, 390 K): 1.62-2.12 (m, 4H, CH₂), 1.94 (s, 3H, OAc), 2.63-2.91 (m, 2H, CH₂Ar), 3.30 (s, 3H, OMe), 3.73 (dd, 1 H, CH or CH₂-OMe), 3.90 (dd, 1 H, CH or CH₂-OMe), 4.31 (d, 2H, CH₂Ph), 4.47 (dd, 1 H, CH or CH₂-OMe), 5.12 (dd, 1 H, ArCH), 6.90-7.35 (m, 9H, Ar-H), 7.95 (bs, 1 H, NH); LC-MS: MH⁺ 381.

### Example 11: (R)-2-{Acetyl-[(R)-1-(4-methoxy-phenyl)-ethyl]-amino}-N-benzyl-3-methoxy-propionamide Id (a compound of the formula I, R1 = (R)-1-(4-methoxy-phenyl)-ethyl)

12.3 g (80.5 mmol) of (R)-1-(4-Methoxyphenyl)ethylamine (a compound of the formula X, R1 = (R)-1-(4-methoxy-phenyl)-ethyl) and 5.3 g (87 mmol) acetic acid (in 50 ml MeOH/water 10:1), 9.62 g (80.5 mmol) benzylisonitrile (in 50 ml MeOH) and 50 ml (95 mmol) methoxyacetaldehyde (1.9 M in water), were allowed to react for 60 min as described in general procedure 3 at 60°C. HPLC (Merck Chromolith Performance RP18e, A. H₂O/0.05% TFA, B: MeCN/0.05% TFA, 10->70% B 10 min, 4 ml/min, 40°C) revealed almost complete consumption of starting material with diastereomeric ratio of 45:55 ((R,R):(R,S)).
60 ml water and 30 ml MeOH were added, the mixture was allowed to cool to 40°C and seed crystals of the (R,R)-diastereomer.were added. The mixture was allowed to cool to rt over night, was then cooled to 0°C and the solid was filtered to yield 9.8 g (32%) of the pure title compound Id with 98% de. The analytical data were identical with those obtained in example 1.

### Example 12: 2-(Acetyl-triphenylmethyl-amino)-N-benzyl-3-methoxy-propionamide (a compound of the formula I, R1 = Triphenylmethyl)

2.13 g (8.1 mmol) of triphenylmethylamine (a compound of the formula X, R1 = Triphenylmethyl), 0.50 g (8.3 mmol) acetic acid, 0.96 g (8.1 mmol) benzylisonitrile and 0.70 g (9.5 mmol) methoxyacetaldehyde in 5 ml MeOH/water 1:1, were allowed to react for 16 hours as described in general procedure 2 to yield 3.89 g (98 %) of the crude title compound. 2.11 g (53 %) of the pure compound were obtained after column chromatography on silica gel with heptane/ethyl acetate 4:1 → 1:2.
Chiral HPLC (Chiralcel OD-H/126 (250x4.6 mm), Heptane:EtOH:MeOH 20:1:1 +0.1% TFA, 30°C, 1 ml/min): Rₜ = 5.92 min (ent1), 8.01 min (ent2); HPLC (Merck Chromolith Performance RP18e, A. H₂O/0.05% TFA, B: MeCN/0.05% TFA, 10->70% B 10 min, 4 ml/min, 40°C): Rₜ = 5.60 min; NMR (250 MHz, 390 K): 1.55 (s, 3H, Ac), 3.19 (s, 3H, OMe), 3.63-3.67 (m, 1 H, CH or CH₂-OMe), 4.17-4.28 (m, 2H, CH or CH₂-OMe), 4.37-4.39 (d, 2H, CH₂Ph), 7.27-7.39 (m, 15H, Ar-H), 7.49-7.54 (m, 6H, Ar-H), 7.82 (s, 1 H, NH); LC-MS: MH⁺ 243 (Trityl).

### Example 13: 2-(Acetyl-benzhydryl-amino)-N-benzyl-3-methoxy-propionamide (a compound of the formula I, R1 = Benzhydryl)

1.52 g (8.1 mmol) of benzhydrylamine (a compound of the formula X, R1 = Benzhydryl), 0.50 g (8.3 mmol) acetic acid, 0.96 g (8.1 mmol) benzylisonitrile and 0.70 g (9.5 mmol) methoxyacetaldehyde in 5 ml MeOH/water 1:1, were allowed to react for 16 hours as described in general procedure 2 to yield 3.36 g (100 %) of the crude title compound. 2.64 g (79 %) of the pure compound were obtained after column chromatography on silica gel with heptane/ethyl acetate 2:1 -> 1:2. Chiral HPLC (Chiralcel OD-H/126 (250x4.6 mm), Heptane:iPrOH:MeOH 20:1:1 +0.1% TFA, 30°C, 1 ml/min): Rₜ = 8.02 min (ent1), 8.81 min (ent2); HPLC (Merck Chromolith Performance RP18e, A. H₂O/0.05% TFA, B: MeCN/0.05% TFA, 10->70% B 10 min, 4 ml/min, 40°C): Rₜ = 4.58 min; NMR (250 MHz, d6-DMSO, 390 K): 2.03 (s, 3H, Ac), 3.15 (s, 3H, OMe), 3.56-3.62 (m, 1 H), 3.90-3.97 (m, 1 H), 4.19-4.37 (m, 2H), 4.38-4.43 (t, 1 H, CH), 6.38 (s, 1 H, CH-Ph₂), 7.23-7.44 (m, 15H, Ar-H), 7.81 (s, 1 H, NH); LC-MS: MH⁺ 417 (167).

### Example 14: 2-(Acetyl-benzyl-amino)-N-benzyl-3-methoxy-propionamide (a compound of the formula I, R1 = Benzyl)

0.87 g (8.1 mmol) of benzylamine (a compound of the formula X, R1 = Benzyl), 0.50 g (8.3 mmol) acetic acid, 0.96 g (8.1 mmol) benzylisonitrile and 0.70 g (9.5 mmol) methoxyacetaldehyde in 5 ml MeOH/water 1:1, were allowed to react for 16 hours as described in general procedure 2 to yield 2.68 g (98 %) of crude title compound. 2.17 g (79 %) of the pure compound were obtained after column chromatography on silica gel with heptane/ethyl acetate 1:1 -> 1:2.
Chiral HPLC (Chiralcel OD-H/126 (250x4.6 mm), Heptane:iPrOH:MeOH 20:1:1 +0.1% TFA, 30°C, 1 ml/min): Rₜ = 10.60 min (ent1), 11.76 min (ent2); HPLC (Merck Chromolith Performance RP18e, A. H₂O/0.05% TFA, B: MeCN/0.05% TFA, 10->70% B 10 min, 4 ml/min, 40°C): Rₜ = 3.27 min; NMR (250 MHz, 390 K): 2.09 (s, 3H, Ac), 3.19 (s, 3H, OMe), 3.58-3.65 (m, 1 H), 3.72-3.79 (m, 1 H), 4.28-4.33 (m, 2H), 4.54-4.60 (m, 1 H) 4.76-4.82 (m, 1 H), 4.87-4.91 (m, 1 H), 7.22-7.38 (m, 10H, Ar-H), 8.00 (s, 1 H, NH); LC-MS: MH⁺ 341 (234).

### Example 15: 2-[Acetyl-(4-methoxy-benzyl)-amino]-N-benzyl-3-methoxy-propionamide (a compound of the formula I, R1 = 4-Methoxy-benzyl)

5.63 g (40.2 mmol) of 4-methoxy-benzyl amine (a compound of the formula X, R1 = 4-Methoxy-benzyl), 2.52 g (41.5 mmol) acetic acid, 4.81 g (40.2 mmol) benzylisonitrile and 3.52 g (47.5 mmol) methoxyacetaldehyde in 25 ml MeOH/water 1:1, were allowed to react for 16 hours as described in general procedure 1 to yield 15.72 g (>100 %) of crude title compound. 12.19 g (82 %) of the pure compound were obtained after column chromatography on silica gel with heptane/ethyl acetate 1:1 -> 1:9. Chiral HPLC (Chiralcel OD-H/126 (250x4.6 mm), Heptane:iPrOH:MeOH 20:1:1 +0.1 % TFA, 30°C, 1 ml/min): Rₜ = 14.04 min (ent1), 15.29 min (ent2); HPLC (Merck Chromolith Performance RP18e, A. H₂O/0.05% TFA, B: MeCN/0.05% TFA, 10->70% B 10 min, 4 ml/min, 40°C): Rₜ = 3.25 min; NMR (250 MHz, 390 K): 2.08 (s, 3H, Ac), 3.21 (s, 3H, OMe), 3.58-3.65 (m, 1 H), 3.72-3.78 (m, 1 H), 3.80 (s, 3H, Ph-OMe), 4.29-4.33 (m, 2H), 4.46-4.53 (m, 1 H) 4.67-4.74 (m, 1 H), 4.82-4.87 (m, 1 H), 6.86-6.92 (d, 2H, Ar-H), 7.20-7.39 (m, 7H, Ar-H), 7.94 (s, 1 H, NH); LC-MS: MH⁺ 371 (264, 121).
General procedure 4 for preparation of compounds of (R)-2-Acetylamino-N-benzyl-3-methoxy-propionamide ((R)-Lacosamide, formula II) according to method 4:

A compound of formula I was dissolved in formic acid and stirred for the appropriate time and temperature. The mixture was extracted twice with toluene. The formic acid layer was concentrated and the residue was purified on silica or crystallised from an appropriate solvent.

### Example 16: (R)-Lacosamide (formula II) from diastereomeric enriched (R)-2-{Acetyl-[(S)-1-(4-methoxy-phenyl)-ethyl]-amino}-N-benzyl-3-methoxy-propionamide Ib by method 4.

8 g (88:12 (S,R):(S,S):diastereomeric mixture) of (R)-2-{Acetyl-[(S)-1-(4-methoxyphenyl)-ethyl]-amino}-N-benzyl-3-methoxy-propionamide Ib (a compound of the formula I, R1 = (S)-1-(4-methoxy-phenyl)-ethyl) was allowed to react in 50 ml formic acid at 70°C for 1 h according to general procedure 4. The crude title compound was crystallised twice from 50 ml toluene and 20 ml AcOEt to yield 1.8 g of (R)-Lacosamide as a colourless solid with 99% ee. Chiral HPLC (Chiralpak AD-H/91 (250x4.6 mm), EtOH, 30°C, 1 ml/min): Rₜ = 13.7 min; HPLC (Merck Chromolith Performance RP18e, A. H₂O/0.05% TFA, B: MeCN/0.05% TFA, 10->70% B 10 min, 4 ml/min, 40°C): Rₜ = 1.15 min; NMR (500 MHz, 300 K): 1.87 (s, 3H, Ac), 3.25 (s, 3H, OMe), 3.49 (dd, 1 H, CH₂-OMe), 3.53 (dd, 1 H, CH₂-OMe), 4.29 (d, 2H, CH₂Ph), 4.46-4.53 (m, 1 H, CH), 72.20-7.35 (m, 5H, Ar-H), 8.09 (d, 1H, NH), 8.49 (t, 1 H, NH); LC-MS: MH⁺ 251.

### Example 17: (R)-Lacosamide (formula II) from (R)-2-{Acetyl-[(R)-1-(4-methoxyphenyl)-ethyl]-amino}-N-benzyl-3-methoxy-propionamide Id by method 4.

400 mg of (R)-2-{Acetyl-[(S)-1-(4-methoxy-phenyl)-ethyl]-amino}-N-benzyl-3-methoxy-propionamide Id (a compound of the formula I, R1 = (R)-1-(4-methoxy-phenyl)-ethyl) was allowed to react in 2.5 ml formic acid at 70°C for 2 h according to general procedure 4. The crude title compound was crystallised from 5 ml MIBK to yield 150 mg (54%) of (R)-Lacosamide as a colourless solid with 99% ee. The analytical data were identical with those obtained in example 16.

General procedure 5 for preparation of compounds of (R)-2-Acetylamino-N-benzyl-3-methoxy-propionamide ((R)-Lacosamide, formula II) according to method 5:
A compound of formula I was dissolved in an appropriate solvent, catalyst was added and the mixture was allowed stirring for the appropriate time and temperature. The catalyst was removed by filtration and the mixture was concentrated. The crude (R)-Lacosamide was purified on silica or crystallised from an appropriate solvent.

### Example 18: (R,S)-Lacosamide (formula II) from (R,S)-2-[Acetyl-((S)-1-naphthalen-2-yl-ethyl)-amino]-N-benzyl-3-methoxy-propionamide by method 5.

360 mg of (R,S)-2-[Acetyl-((S)-1-naphthalen-2-yl-ethyl)-amino]-N-benzyl-3-methoxy-propionamide (a compound of the formula I, R1 = (S)-1-(naphthalen-2-yl)-ethyl) 60/40 diastereomeric mixture, and 50 mg PdOH₂ on carbon (20%) in 10 ml acetic acid were allowed to react room temperature for 7d according to general procedure 5. LCMS revealed about 10% of Lacosamide. LC: (YMC J' sphere ODS H80, A. H₂O/0.05% TFA, B: MeCN, 4->95% B 2 min, 1 ml/min, 30°C): Rₜ = 0.76 min; MS: MH⁺ 251.

### Example 19: (R)-Lacosamide (formula ll) from ((R)-2-{Acetyl-[(R)-1-(4-methoxy-phenyl)-ethyl]-amino}-N-benzyl-3-methoxy-propionamide Id by procedure 5.

100 mg of ((R)-2-{Acetyl-[(R)-1-(4-methoxy-phenyl)-ethyl]-amino}-N-benzyl-3-methoxy-propionamide Id and 1 equivalent of BF₃xOEt₂ in 2ml dichloromethane were allowed to react for 1 h at rt. LCMS showed almost complete consumption of starting material (> 96%). LC: (YMC J' sphere ODS H80, A. H₂O/0.05% TFA, B: MeCN, 4->95% B 2 min, 1 ml/min, 30°C): Rₜ = 0.73 min; MS: MH⁺ 251.

## Claims

1. A compound of formula I, enantiomerically and diastereomerically pure or as mixture with the corresponding enantiomer or diastereomer, whereas formula I represents both the pure enantiomer or diastereomer as well as its mixture with the corresponding enantiomer or diastereomer, 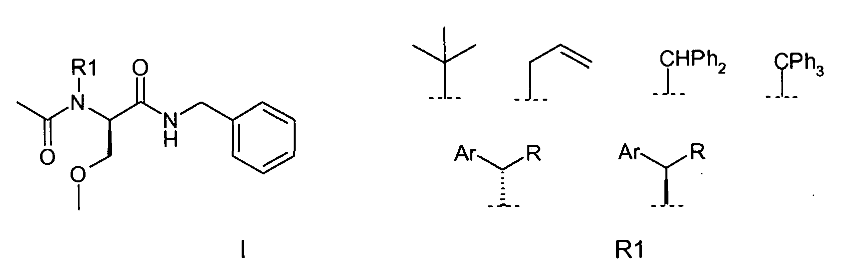 wherein
R1 is allyl-, tert-butyl-, di- and triphenylmethyl 1-naphthalen-2-yl-ethyl, 1-naphthalen-1-yl-ethyl or
a 1-aryl-1-R-methyl group where
aryl (Ar) is a phenyl group, optionally substituted by one, two or three residues independently selected from (C₁-C₄)alkyl, (C₁-C₄)alkoxy, halogen or nitro; and
R is H or a (C₁-C₄)alkyl group with n = 1-4 carbon atoms - wherein for n = 2, 3 or 4 R can be connected to the aryl group to form an aromatic or partially unsaturated C₆-C₁₀ bicyclic residue.

2. A process for preparing a compound of the formula I, enantiomerically and diastereomerically pure or as mixture with the corresponding enantiomer or diastereomer, whereas formula I represents both the pure enantiomer or diastereomer as well as its mixture with the corresponding enantiomer or diastereomer, 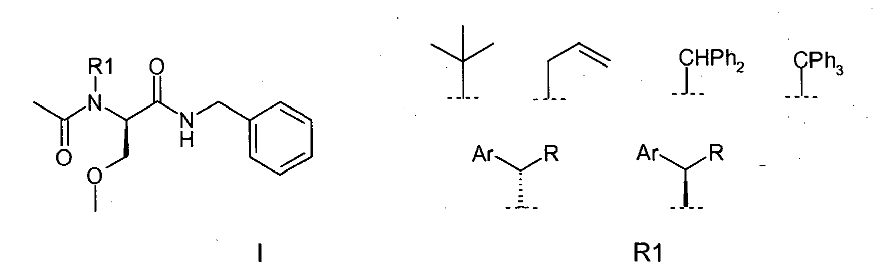 which comprises 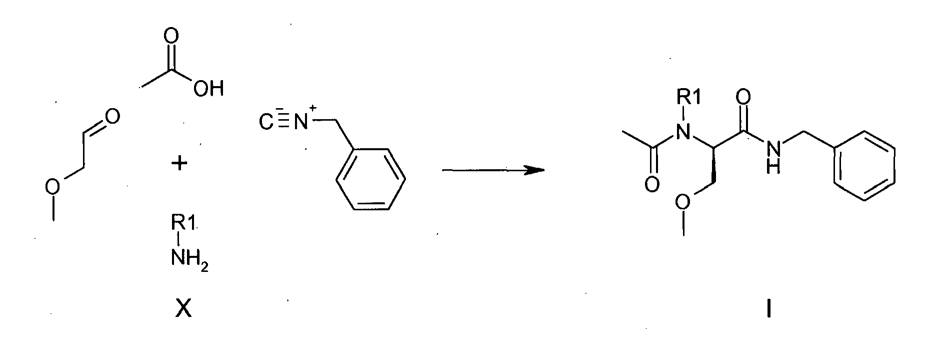 reacting a compound R1-NH2 (X) with acetic acid, Methoxyacetaldehyde and Benzylisonitrile, wherein R1 is as defined in formula I of claim 1.

3. The process as claimed in claim 2, wherein
an amine R1 NH2 (X) where R1 is defined as in formula I, is added to a mixture of acetic acid (VII), methoxyacetaldehyde (VIII) and benzylisonitrile (IX).

4. The process as claimed in claim 2, wherein
methoxyacetaldehyde (VIII) is added to a mixture of an amine of formula R1 NH2 (X) wherein R1 is defined as in formula I, acetic acid and benzylisonitrile.

5. The process as claimed in claim 2, wherein
a salt, prepared by mixing a compound of formula X and acetic acid, and methoxyacetaldehyde are added simultaneously or successively to benzylisonitrile.

6. The process as claimed in claims 2 to 5, wherein
compound R1 NH2 (X), acetic acid, methoxyacetaldehyde and benzylisonitrile are mixed in a continuous flow reactor.

7. The process as claimed in claims 2 to 6, wherein
R1 is a 1-aryl-1-R-methyl group - where aryl (Ar) is a phenyl group, optionally substituted by one, two or three residues selected from (C₁-C₄)alkyl, (C₁-C₄)alkoxy, halogen or nitro; and R is H or a (C₁-C₄)alkyl group with n = 1-4 carbon atoms - wherein for n = 2,3,4 R can be connected to the aryl group to form a aromatic or partially saturated C₆-C₁₀ bicyclic residue.

8. The process as claimed in claims 2 to 7, wherein
R1 is selected from 1-(4-methoxy-phenyl)-ethyl, 1-phenyl-ethyl, 1-phenyl-propyl, 1-naphthalen-2-yl-ethyl, 1-naphthalen-1-yl-ethyl, 1-(4-methyl-phenyl)-ethyl, 1-(2-methoxy-phenyl)-ethyl, 1-(4-nitro-phenyl)-ethyl, indan-1-yl, or 1-(1,2,3,4-tetrahydro-naphthalen-1-yl)-ethyl, which group is of either R or S-configuration or a mixture thereof,

9. The process as claimed in claims 2 to 6, wherein
R1 is benzyl, 2-methoxy benzyl, 3-methoxy benzyl, 4-methoxy benzyl, 3,4-dimethoxy benzyl, allyl, tert-butyl, diphenylmethyl or triphenylmethyl.

10. The process as claimed in claims 2 to 9, wherein
a compound of formula I is directly obtained from the reaction mixture by crystallisation.

11. The process as claimed in claims 2 to 9, wherein a compound of formula I is obtained from the reaction by chromatography of the product mixture, optionally on a chiral stationary phase.

12. Use of compound of formula I 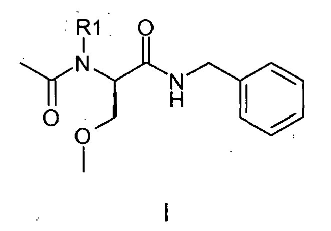 wherein R1 is defined as in claim 1,
for the preparation of (R)-Lacosamide II

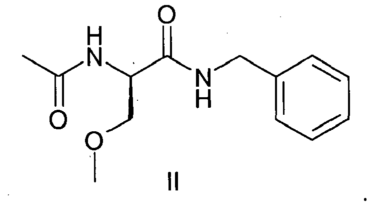

13. A process for preparing (R)-Lacosamide of the formula II
which comprises removing R1 in compound I 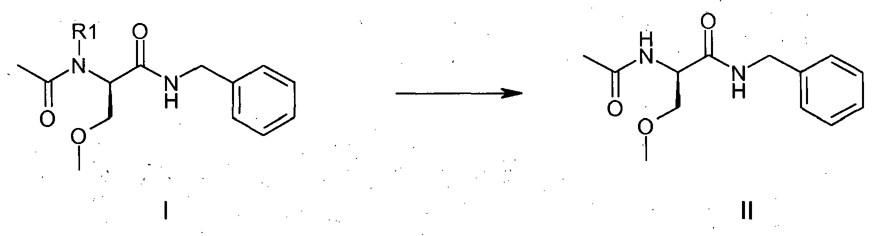 wherein R1 in compound I is defined as in claim 1
with a suitable reagent, optionally in a suitable solvent.

14. The process as claimed in claim 13, wherein an acid is used as reagent.

15. The process as claimed in claim 13 or 14, wherein
the acid is formic acid, acetic acid, trifluoroacetic acid, trichloroacetic acid, methane sulfonic acid, trifluoromethane sulfonic acid, HBr, HCl, HI, H₂SO₄ or H₃PO₄ or a mixture of at least 2 of the aforesaid acids.

16. The process as claimed in claim 13 wherein a Lewis acid is used as reagent.

17. The process as claimed in claim 13 or 16, wherein
the Lewis acid is boron trifluoride, titanium tetrachloride, aluminum trichloride, ferric chloride or zinc chloride or a mixture of at least 2 of aforesaid Lewis acids.

18. The process as claimed in claim 13, wherein a hydrogen source and a transition metal are used as reagent.

19. The process as claimed in claim 13 or 18, wherein
the hydrogen source is hydrogen, formic acid and their salts, cyclohexadiene or a mixture of at least 2 of aforesaid hydrogen sources.

20. The process as claimed in claim 13 or 18 wherein the transition metal is Palladium, Rhodium, Platinum or Iridium.

21. The process as claimed in claim 13 or 18 wherein an acid is added selected from acetic acid, 2-chloro- acetic acid, methoxy-acetic acid, propionic acid, butyric acid, valeric acid or pivalic acid or a mixture of at least 2 of aforesaid acids.

22. A process for preparing (R)-Lacosamide II, which comprises 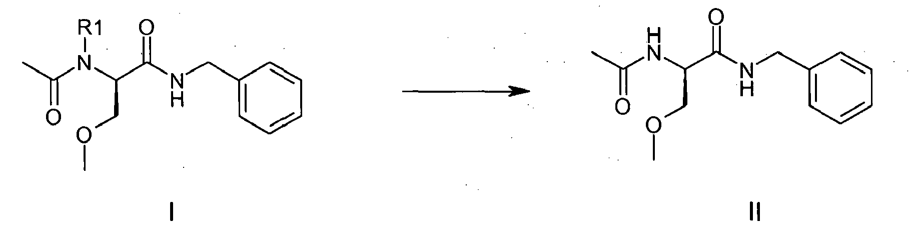 removing R1 in a compound-of formula I, wherein R1 is allyl (CH2CH=CH2) and I is enantiomerically pure or in a mixture with its enantiomer.

23. The process a claimed in claim 22 wherein Palladium or Rhodium is used.

## Patentansprüche

1. Verbindung der Formel I, enantiomerenrein und diastereomerenrein oder als Mischung mit dem entsprechenden Enantiomer oder Diastereomer, wobei Formel I sowohl für das reine Enantiomer oder Diastereomer als auch für dessen Mischung mit dem entsprechenden Enantiomer oder Diastereomer steht, 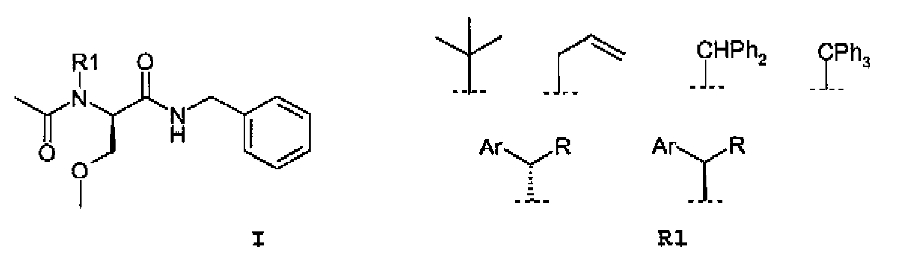 wobei
R1 Allyl-, tert-Butyl-, Di- und Triphenylmethyl, 1-Naphthalen-2-yl-ethyl, 1-Naphthalen-1-yl-ethyl oder
eine 1-Aryl-1-R-Methyl-Gruppe ist, wobei
Aryl (Ar) eine Phenylgruppe ist, optional substituiert durch ein, zwei oder drei Reste, die unabhängig voneinander aus (C₁-C₄)-Alkyl, (C₁-C₄)-Alkoxy, Halogen oder Nitro ausgewählt sind; und
R H oder eine (C₁-C₄) -Alkylgruppe mit n = 1-4 Kohlenstoffatomen ist - wobei, wenn n = 2, 3 oder 4, so kann R an die Arylgruppe gebunden sein, so dass ein aromatischer oder partiell ungesättigte bicyclischer (C₆-C₁₀)-Rest gebildet wird.

2. Ein Verfahren zur Herstellung einer Verbindung der Formel I, in enantiomerenreiner und diastereomerenreiner Form oder als Mischung mit dem entsprechenden Enantiomer oder Diastereomer, wobei Formel I sowohl für das reine Enantiomer oder Diastereomer als auch für dessen Mischung mit dem entsprechenden Enantiomer oder Diastereomer steht, 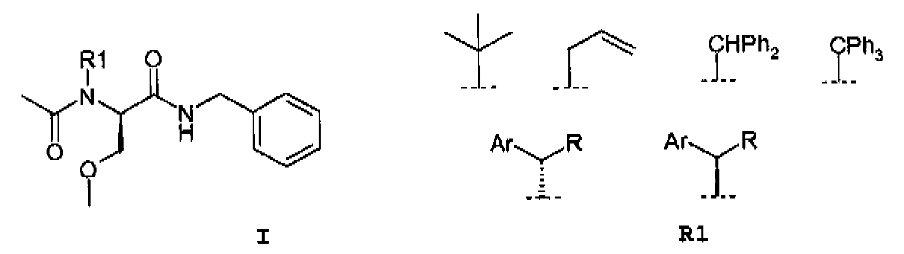 welches umfasst : 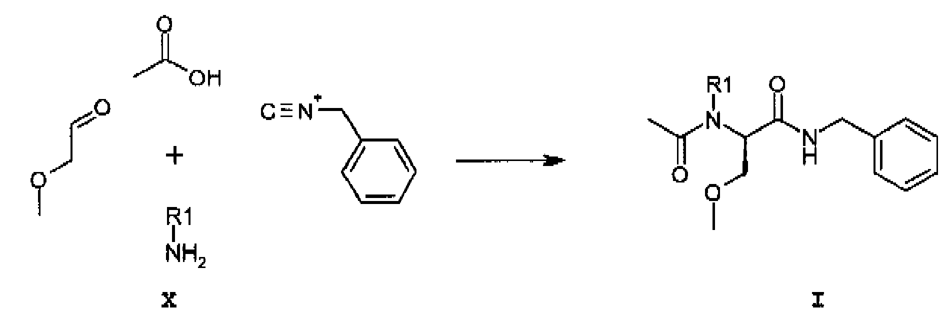 Umsetzen einer Verbindung R1-NH2 (X) mit Essigsäure, Methoxyacetaldehyd und Benzylisonitril, wobei R1 wie in Formel I des Anspruchs 1 definiert ist.

3. Verfahren nach Anspruch 2, bei dem
ein Amin R1NH2 (X), wobei R1 wie in Formel I definiert ist, zu einem Gemisch aus Essigsäure (VII), Methoxyacetaldehyd (VIII) und Benzylisonitril (IX) hinzugefügt wird.

4. Verfahren nach Anspruch 2, bei dem
Methoxyacetaldehyd (VIII) zu einem Gemisch aus einem Amin der Formel R1NH2 (X), wobei R1 wie in Formel I definiert ist, Essigsäure und Benzylisonitril hinzugegeben wird.

5. Verfahren nach Anspruch 2, bei dem
ein Salz, hergestellt durch Mischen einer Verbindung der Formel X und Essigsäure, und
Methoxyacetaldehyd gleichzeitig oder nacheinander zu Benzylisonitrile hinzugegeben werden.

6. Verfahren nach Ansprüche 2 bis 5, bei dem die Verbindung R1NH2 (X), Essigsäure, Methoxyacetaldehyd und Benzylisonitril in einem Durchflussreaktor gemischt werden.

7. Verfahren nach Ansprüchen 2 bis 6, bei dem R1 eine 1-Aryl-1-R-Methyl-Gruppe ist - wobei Aryl (Ar) eine Phenylgruppe ist, optional substituiert durch ein, zwei oder drei Reste, die aus (C₁-C₄)-Alkyl, (C₁-C₄)-Alkoxy, Halogen oder Nitro ausgewählt sind; und R H ist oder eine (C₁-C₄)-Alkylgruppe mit n = 1-4 Kohlenstoffatomen - wobei, wenn n = 2,3,4, R an die Arylgruppe gebunden sein kann, sodass ein aromatischer oder partiell gesättigter bicyclischer C₆-C₁₀-Rest gebildet wird.

8. Verfahren nach Ansprüche 2 bis 7, bei dem R1 aus 1-(4-Methoxy-phenyl)-ethyl, 1-Phenyl-ethyl, 1-Phenyl-propyl, 1-Naphthalen-2-yl-ethyl, 1-Naphthalen-1-yl-ethyl, 1-(4-Methyl-phenyl)-ethyl, 1-(2-Methoxy-phenyl)-ethyl, 1-(4-Nitro-phenyl)-ethyl, Indan-1-yl oder 1-(1,2,3,4-Tetrahydro-naphthalen-1-yl)-ethyl, wobei die Gruppe entweder R- oder S-Konfiguration aufweiset, oder einer Mischung davon, ausgewählt ist.

9. Verfahren nach Ansprüchen 2 bis 6, bei dem
R1 Benzyl, 2-Methoxybenzyl, 3-Methoxybenzyl, 4-Methoxybenzyl, 3,4-Dimethoxybenzyl, Allyl, tert-Butyl, Diphenylmethyl oder Triphenylmethyl ist.

10. Verfahren nach Anspruch 2 bis 9, bei dem
eine Verbindung der Formel I direkt aus dem Reaktionsgemisch durch Kristallisation erhalten wird.

11. Verfahren nach den Ansprüchen 2 bis 9, bei dem eine Verbindung der Formel I aus der Reaktion durch Chromatographie des Produktgemisches, walweise auf einer chiralen stationären Phase, erhalten wird.

12. Verwendung der Verbindung der Formel I 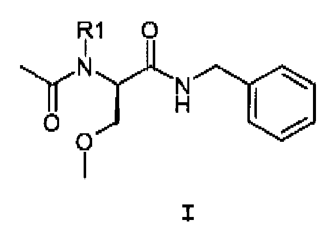 wobei R1 wie in Anspruch 1 definiert ist,
für die Herstellung von (R)-Lacosamid II

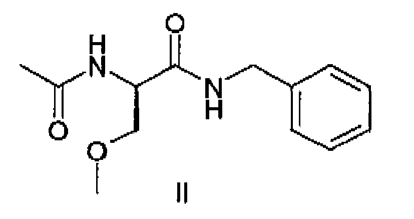

13. Verfahren zur Herstellung von (R)-Lacosamid der Formel II, welches das Entfernen von R1 in der Verbindung I umfasst. 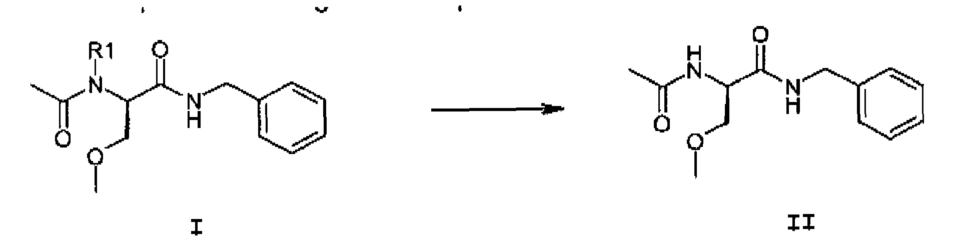 wobei R1 in der Verbindung I wie in Anspruch 1 definiert ist mit einem geeigneten Reagens, wahlweise in einem geeigneten Lösungsmittel.

14. Verfahren nach Anspruch 13, bei dem eine säure als Reagens verwendet wird.

15. Verfahren nach Anspruch 13 oder 14, bei dem es sich bei der Säure um Ameisensäure, Essigsäure, Trifluoressigsäure, Trichloressigsäure, Methansulfonsäure, Trifluormethansulfonsäure, HBr, HCl, HI, H₂SO₄ oder H₃PO₄, oder um ein Gemisch aus wenigstens 2 der vorgenannten Säure, handelt.

16. Verfahren nach Anspruch 13, bei dem eine Lewis-Säure als Reagens verwendet wird.

17. Verfahren nach Anspruch 13 oder 16, bei dem die Lewis-Säure Bortrifluorid, Titantetrachlorid, Aluminiumtrichlorid, Ferrichlorid oder Zinkchlorid, oder ein Gemisch aus wenigstens 2 der vorgenannten Lewis-Säuren, ist.

18. Verfahren nach Anspruch 13, bei dem eine Wasserstoffquelle und ein Übergangsmetall als Reagens verwendet werden.

19. Verfahren nach Anspruch 13 oder 18, bei dem die Wasserstoffquelle Wasserstoff, Ameisensäure und deren Salze, Cyclohexadien, oder ein Gemisch aus wenigstens 2 der vorgenannten Wasserstoffquellen, ist.

20. Verfahren nach Anspruch 13 oder 18, bei dem das Übergangsmetall Palladium, Rhodium, Platin oder Iridium ist.

21. Verfahren nach Anspruch 13 oder 18, bei dem eine Säure hinzugegeben wird, die aus Essigsäure, 2-Chloressigsäure, Methoxyessigsäure, Propionsäure, Buttersäure, Valeriansäure oder Pivalinsäure, oder einem Gemisch aus wenigstens 2 der oben genannten Säuren, ausgewählt ist.

22. Verfahren zur Herstellung von (R)-Lacosamid II, welches umfasst : 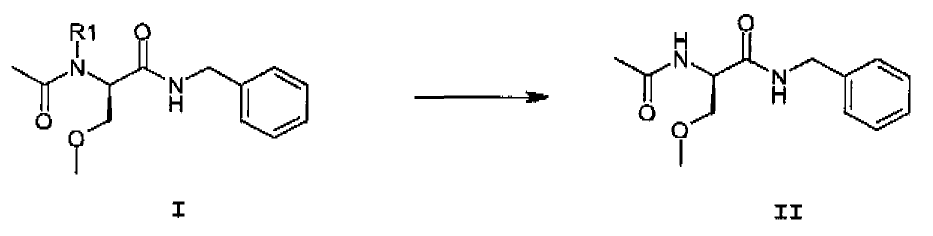 Entfernen von R1 in einer Verbindung der Formel I, wobei R1 Allyl (CH2CH=CH2) und I enantiomerenrein ist oder in einer Mischung mit seinem Enantiomer vorliegt.

23. Verfahren nach Anspruch 22, bei dem Palladium oder Rhodium verwendet wird.

## Revendications

1. Un composé de formule I, énantiomériquement et diastéréoisomériquement pur ou sous forme de mélanges avec l'énantiomère ou le diastéréoisomère correspondant, la formule I représentant à la fois l'énantiomère pur ou le diastéréoisomère pur ainsi que leurs mélanges avec l'énantiomère ou le diastéréoisomère correspondant, 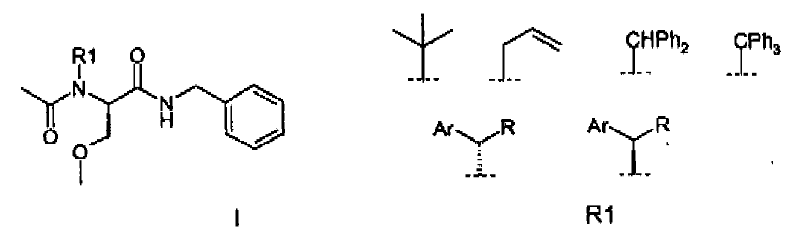 où
R1 représente un groupe allyl, tert-butyl, di- ou triphénylméthyl, 1-naphtalène-2-yl-éthyl, 1-naphtalène-1-yl-éthyl ou 1-aryl-1-R-méthyl où
- aryl (Ar) est un groupe phényl, éventuellement substitué par un, deux ou trois résidus choisis indépendamment les uns des autres parmi (C₁-C₄)-alkyl, (C₁-C₄)-alkoxy, les atomes d'halogène ou un groupe nitro; et
- R est un atome d'hydrogène ou un groupe (C₁-C₄)alkyl avec n = 1 -4 atomes de carbone - où quand n = 2, 3 ou 4, les R peuvent être reliés au groupe aryl pour former un résidu C₆-C₁₀ bicyclique aromatique ou partiellement insaturé.

2. Un procédé de préparation d'un composé de formule I, énantiomériquement et diastéréoisomériquement pur ou sous forme de mélanges avec l'énantiomère ou le diastéréoisomère correspondant, la formule I représentant à la fois l'énantiomère pur ou le diastéréoisomère pur ainsi que leurs mélanges avec l'énantiomère ou le diastéréoisomère correspondant, 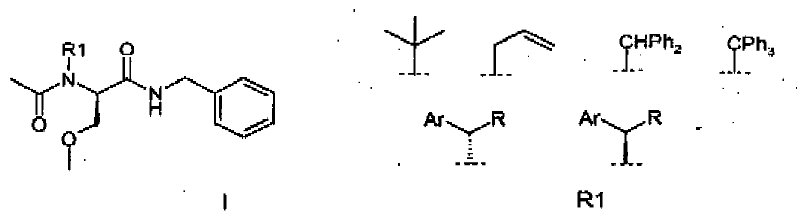 qui comprend 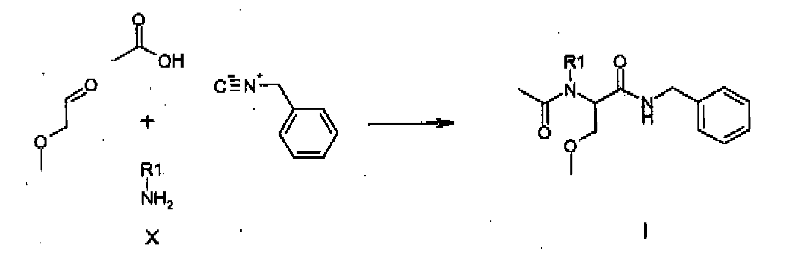 la réaction d'un composé R1-NH2 (X) avec de l'acide acétique, du méthoxyacétaldéhyde et du benzylisonitrile, où R1 est tel que défini dans la formule I de la revendication 1.

3. Le procédé tel que revendiqué dans la revendication 2, dans lequel une amine R1NH2 (X) dans laquelle R1 est défini comme dans la formule I, est ajoutée à un mélange d'acide acétique (VII), de méthoxyacétaldéhyde (VIII) et de benzylisonitrile (IX).

4. Le procédé tel que revendiqué dans la revendication 2, dans lequel le méthoxyacétaldéhyde (VIII) est ajouté à un mélange d'une amine de formule RINH2 (X) dans laquelle R1 est défini comme dans la formule I, d'acide acétique et de benzylisonitrile.

5. Le procédé tel que revendiqué dans la revendication 2, dans lequel un sel, préparé en mélangeant un composé de formule X et de l'acide acétique, et le méthoxyacétaldéhyde sont ajoutés simultanément ou successivement au benzylisonitrile.

6. Le procédé tel que revendiqué dans les revendications 2 à 5, dans lequel le composé R1NH2 (X), l'acide acétique, le méthoxyacétaldéhyde et le benzylisonitrile sont mélangés dans un réacteur à écoulement continu.

7. Le procédé tel que revendiqué dans les revendications 2 à 6, dans lequel
- R1 est un groupe 1-aryl-1-R-méthyl - où le groupe aryl (Ar) est un groupe phényl, éventuellement substitué par un, deux ou trois résidus choisis parmi les (C₁-C₄)-alkyl, (C₁-C₄) alkoxy, les atomes d'halogène ou un groupe nitro; et
- R est un atome d'hydrogène ou un groupe (C1-C4) alkyl où n = 1 -4 atomes de carbone - dans lequel quand n = 2,3,4 alors R peut être lié au groupe aryl pour former un résidu C₆-C₁₀ bicyclique aromatique ou partiellement saturé.

8. Le procédé tel que revendiqué dans les revendications 2 à 7, dans lequel
- R1 est choisi parmi les 1-(4-méthoxy-phényl)-éthyl, 1-phényl-éthyl, 1-phényl-propyl, 1-naphtalène-2-yl-éthyl, 1 -naphtalène-1 -yl-éthy 1, 1-(4-méthyl-phényl)-éthyl, 1-(2-méthoxy-phényl)-éthyl, 1-(4-nitro-phényl)-éthyl, indan-1-yl ou 1-(1,2,3,4-tétrahydro-naphtalène-1-yl)-éthyl, lequel groupe est soit en configuration R soit en configuration S ou en un mélange de ceux-ci,

9. Le procédé tel que revendiqué dans les revendications 2 à 6, dans lequel R1 est un groupe benzyl, 2-méthoxy benzyl, 3-méthoxy benzyl, 4-méthoxy benzyl, 3,4-diméthoxy-benzyl, allyl, tert-butyl, diphénylméthyl ou triphénylméthyl.

10. Le procédé tel que revendiqué dans les revendications 2 à 9, dans lequel un composé de formule I est obtenu directement à partir du mélange réactionnel par cristallisation.

11. Le procédé tel que revendiqué dans les revendications 2 à 9, dans lequel un composé de formule I est obtenu à partir de la réaction par chromatographie du mélange de produits, le cas échéant sur une phase stationnaire chirale.

12. Utilisation d'un composé de formule I 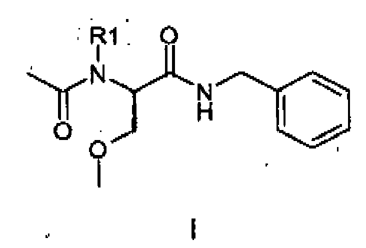 dans laquelle R1 est défini comme dans la revendication 1,
pour la préparation de (R)-Lacosamide II

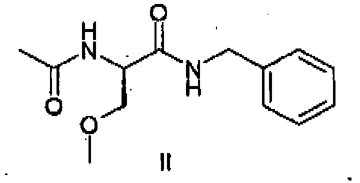

13. Un procédé de préparation de (R)-Lacosamide de formule II qui comprend l'élimination de R1 dans le composé I 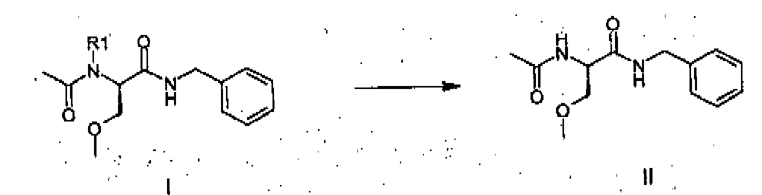 dans lequel R1 dans le composé I est défini comme dans la revendication 1 à l'aide d'un réactif approprié, éventuellement dans un solvant approprié.

14. Le procédé tel que revendiqué dans la revendication 13, dans lequel un acide est utilisé comme réactif.

15. Le procédé tel que revendiqué dans la revendication 13 ou 14, dans lequel l'acide est l'acide formique, l'acide acétique, l'acide trifluoroacétique, l'acide trichloroacétique, l'acide méthanesulfonique, l'acide trifluorométhanesulfonique, HBr, HCl, HI, H₂SO₄ ou H₃PO₄ ou un mélange d'au moins deux des acides précités.

16. Le procédé tel que revendiqué dans la revendication 13, dans lequel un acide de Lewis est utilisé en tant que réactif.

17. Le procédé tel que revendiqué dans la revendication 13 ou 16, dans lequel l'acide de Lewis est le trifluorure de bore, le tétrachlorure de titane, le trichlorure d'aluminium, le chlorure ferrique ou le chlorure de zinc ou un mélange d'au moins deux des acides de Lewis mentionnés ci-dessus.

18. Le procédé tel que revendiqué dans la revendication 13, dans lequel une source d'hydrogène et un métal de transition sont utilisés en tant que réactif.

19. Le procédé tel que revendiqué dans la revendication 13 ou 18, dans lequel la source d'hydrogène consiste en l'hydrogène, l'acide formique et leurs sels, le cyclohexadiène ou un mélange d'au moins deux des sources d'hydrogène mentionnées ci-dessus.

20. Le procédé tel que revendiqué dans la revendication 13 ou 18, dans lequel le métal de transition est le palladium., le rhodium, le platine ou l'iridium.

21. Le procédé tel que revendiqué dans la revendication 13 ou 18, dans lequel on ajoute un acide choisi parmi l'acide acétique, l'acide 2-chloro-acétique, l'acide méthoxyacétique, l'acide propionique, l'acide butyrique, l'acide valérique ou l'acide pivalique ou un mélange d'au moins deux de ces acides précités.

22. Un procédé de préparation de (R)-Lacosamide II, qui comprend 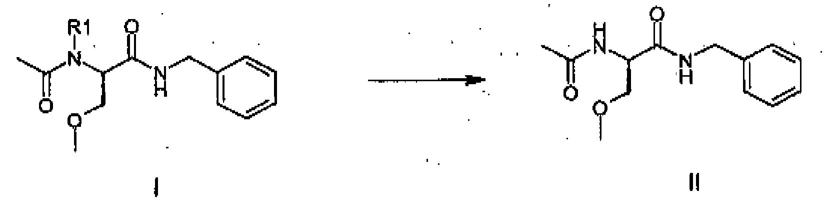 l'enlévement d'un substituant R1 dans un composé de formule I, où R1 est un groupe allyl (CH₂CH=CH₂) et le composé I est énantiomériquement pur ou en mélange avec son énantiomère.

23. Le procédé selon la revendication 22, dans lequel est utilisé le palladium ou le rhodium.
